(19) Europäisches Patentamt
European Patent Office
Office européen des brevets

(11) Publication number: **0 087 856**
**B1**

(12) **EUROPEAN PATENT SPECIFICATION**

(45) Date of publication of patent specification: **26.02.86**

(51) Int. Cl.[4]: **B 01 D 53/34,** B 01 D 53/14, C 07 C 93/04, C 07 D 295/08

(21) Application number: **83300186.0**

(22) Date of filing: **14.01.83**

(54) **A process for the removal of H2S from gaseous mixtures using diaminoethers.**

(30) Priority: **18.01.82 US 339883**
**18.01.82 US 339884**
**18.01.82 US 339890**
**18.01.82 US 339899**
**18.01.82 US 339900**

(43) Date of publication of application:
**07.09.83 Bulletin 83/36**

(45) Publication of the grant of the patent:
**26.02.86 Bulletin 86/09**

(84) Designated Contracting States:
**BE DE FR GB IT NL**

(56) References cited:
**DE-A-2 134 379**
**DE-A-2 824 908**
**DE-C- 960 191**
**US-A-3 400 157**
**US-A-4 217 238**
**US-A-4 238 206**

(73) Proprietor: **Exxon Research and Engineering Company**
**P.O.Box 390 180 Park Avenue**
**Florham Park New Jersey 07932 (US)**

(72) Inventor: **Stogryn, Eugene Leo**
**3 Sharp Road**
**Edison New Jersey 08817 (US)**
Inventor: **Savage, David William**
**6 Sunset Drive**
**Summit New Jersey 07901 (US)**
Inventor: **Sartori, Guido**
**25 West Price Street**
**Linden New Jersey 07036 (US)**

(74) Representative: **Field, Roger Norton et al**
**ESSO Engineering (Europe) Ltd. Patents & Licences Apex Tower High Street**
**New Malden Surrey KT3 4DJ (GB)**

**EP 0 087 856 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to an improved process for the removal of $H_2S$ from normally gaseous mixtures wherein absorbent solutions containing a class of di-aminoethers are employed. More preferably, this invention is directed to a process for selectively removing $H_2S$ from gaseous mixtures containing $H_2S$ and $CO_2$ employing these absorbent solutions. This invention also relates to a novel class of diaminoethers and their preparation.

The treatment of acid gas mixtures containing, *inter alia,* $CO_2$ and $H_2S$ with aqueous amine solutions in an absorber tower typically results in the simultaneous removal of substantial amounts of both the $CO_2$ and $H_2S$. For example, in one such process generally referred to as the "aqueous amine process", relatively concentrated amine solutions are employed. A recent improvement on this process involves the use of sterically hindered amines to obtain nearly complete removal of acid gases such as $CO_2$ and $H_2S$. This type of process may be used where the partial pressures of the $CO_2$ and related gases are low. Another process often used for specialised applications where the partial pressure of $CO_2$ is extremely high and/or where many acid gases are present, e.g., $H_2S$, COS, $CH_3SH$ and $CS_2$, involves the use of an amine in combination with a physical absorbent, generally referred to as the "nonaqueous solvent process". An improvement on this process involves the use of sterically hindered amines and organic solvents as the physical absorbent.

It is often desirable, however, to treat acid gas mixtures containing both $CO_2$ and $H_2S$ so as to remove the $H_2S$ selectively from the mixture, thereby minimizing removal of the $CO_2$. Selective removal of $H_2S$ results in a relatively high $H_2S/CO_2$ ratio in the separated acid gas which simplifies the conversion of $H_2S$ to elemental sulfur using the Claus process.

The typical reactions of aqueous secondary and tertiary amines with $CO_2$ and $H_2S$ can be represented as follows:

$$H_2S+R_3N\rightleftharpoons R_3NH^++SH^- \quad (1)$$

$$H_2S+R_2NH\rightleftharpoons R_2NH_2^++SH^- \quad (2)$$

$$CO_2+R_3N+H_2O\rightleftharpoons R_3NH^++HCO_3^- \quad (3)$$

$$CO_2+2R_2NH\rightleftharpoons R_2NH_2^++R_2NCOO^- \quad (4)$$

wherein R is an organic radical which may be the same or different and may be substituted with a hydroxyl group. The above reactions are reversible, and the partial pressures of both $CO_2$ and $H_2S$ are thus important in determining the degree to which the above reactions occur.

While selective $H_2S$ removal is applicable to a number of gas treating operations including treatment of hydrocarbon gases from shale pyrolysis, refinery gas and natural gas having a low $H_2S/CO_2$ ratio, it is particularly desirable in the treatment of gases wherein the partial pressure of $H_2S$ is relatively low compared to that of $CO_2$ because the capacity of an amine to absorb $H_2S$ from the latter type gases is very low. Examples of gases with relatively low partial pressures of $H_2S$ include synthetic gases made by coal gasification, sulfur plant tail gas and low-Joule fuel gases encountered in refineries where heavy residual oil is being thermally converted to lower molecular weight liquids and gases.

Solutions of primary and secondary amines such as monoethanolamine (MEA), diethanolamine (DEA), dipropanolamine (DPA), and hydroxyethoxyethylamine (DGA) are known to absorb both $H_2S$ and $CO_2$ gas. Diisopropanolamine (DIPA) is relatively unique among secondary aminoalcohols in that it has been used industrially, alone or with a physical solvent such as sulfolane, for selective removal of $H_2S$ from gases containing $H_2S$ and $CO_2$.

The tertiary amine, methyldiethanolamine (MDEA), has a high degree of selectivity toward $H_2S$ absorption over $CO_2$, but recently it was discovered that aqueous solutions of dialkylmonoalkanolamines, and particularly diethylmonoethanolamine (DEAE), have higher selectivity and capacity for $H_2S$ removal at higher loading levels than MDEA solutions.

It has now been discovered that absorbent solutions of a certain class of amino compounds defined as diaminoethers have a high selectivity for $H_2S$ compared to $CO_2$. These diaminoethers surprisingly maintain their high selectivity at high $H_2S$ and $CO_2$ loadings.

The present invention concerns a process for the removal of $H_2S$ from a normally gaseous stream with an absorbent solution comprising a solvent and a diaminoether wherein both amino groups either are sterically hindered (as hereinafter defined) secondary amino groups or are tertiary amino groups or wherein one amino group is a sterically hindered (as hereinafter defined) secondary amino group and the other amino group is a tertiary amino group.

In a preferred embodiment of the invention the process involves the selective absorption of $H_2S$ from a normally gaseous stream also containing $CO_2$ by contacting the stream with a solution of the diaminoether above described under conditions whereby $H_2S$ is selectively absorbed from the stream, wherein the diaminoether is additionally characterized by having a $pK_a$ value at 20°C greater than 8.6. The absorbent solution may additionally contain water, a physical absorbent, preferably sulfolane, or a mixture thereof.

In a still more preferred embodiment, after the $H_2S$ is selectively absorbed from the stream, the

absorbent solution containing the $H_2S$ is regenerated at least partially and the regenerated solution is recycled for selective absorption of $H_2S$ by recontacting it with the stream. Preferably, the regeneration step is carried out by heating and stripping the solution of any of its absorbed $H_2S$ and more preferably by heating and stripping the solution with steam.

The present invention also relates to a novel class of diaminoethers further defined hereinbelow which are useful in the process for removing $H_2S$ from gaseous streams and to a process for their preparation.

In the drawings:

Figure 1 is a diagrammatic flow sheet illustrating an absorption-regeneration unit for selective removal of $H_2S$ from gaseous streams containing $H_2S$ and $CO_2$.

Figure 2 is a diagrammatic flow sheet illustrating an experimental sparged absorber unit for use in rapid determination of the selectivity of the diaminoether for selective removal of $H_2S$ from gaseous streams containing $H_2S$ and $CO_2$.

Figure 3 graphically illustrates the selectivity for $H_2S$ plotted against the $H_2S$ and $CO_2$ loading for 1,2 - bis - (pyrrolidinylethoxy)ethane (BIS-1) and 1,2 - bis - (piperidinylethoxy)ethane (BIS-PIP) as compared to methyldiethanolamine (MDEA) as control.

Figure 4 graphically illustrates the selectivity for $H_2S$ plotted against the $H_2S$ and $CO_2$ loading for bis - (N - pyrrolidinylethyl)ether (BIS-0), 1,2 - bis - (pyrrolidinylethoxyethoxy)ethane (BIS-2) and 1,2 - bis - (3 - pyrrolidinyl - n - propoxy)ethane (BIS-P).

Figure 5 graphically illustrates the selectivity for $H_2S$ plotted against the $H_2S$ and $CO_2$ loading for 1 - (pyrrolidinylethoxy) - 2 - (t - butylaminoethoxy)ethane (PETBEE) as compared with t - butylaminoethoxyethanol (TBEE) as a comparison and methyldiethanolamine (MDEA) as a control.

Figure 6 graphically illustrates the selectivity for $H_2S$ plotted against the $H_2S$ and $CO_2$ loading for 1,2 - bis - (tertiarybutylaminoethoxy)ethane (BIS-TB) as compared to tertiarybutylaminoethoxyethanol (TBEE), 1,2 - bis - (isopropylaminoethoxy)ethane (BIS-IP), and methyldiethanolamine (MDEA) as control.

The diaminoethers which are generally useful in the process of the present invention include mono- or polyether compounds which may be di-tertiaryaminoethers, disecondaryaminoethers or di-mixed-tertiary- and secondary- aminoethers.

The tertiary amine group(s) of the diaminoethers herein may be acyclic or in the form of a nitrogen heterocycle. If the amine portion is heterocyclic, it may contain one or more rings each typically having 4—9 ring atoms, preferably 4—7 ring atoms, wherein one of the ring atoms is nitrogen. The ring or rings may be substituted or unsubstituted. Furthermore, the rings may be azabicyclic wherein two rings are fused so that they share at least one side and one of the ring atoms is nitrogen. The nitrogen atom forming the tertiary amino portion of such a compound is situated within the bicyclic framework at a bridgehead or nonbridgehead ring position. The oxygen atom of the ether functionality may be directly attached to the ring or may be separated from the ring by a hydrocarbon chain, arranged in a linear or branched fashion, which is attached to the ring via a ring nitrogen or a ring carbon atom.

The secondary amine group(s) of the diaminoethers herein is a severely sterically hindered secondary amino group. By the term "severely sterically hindered" it is meant that the nitrogen atom of the amine is attached to one or more bulky carbon groupings. This means that the secondary amine group(s) will have a degree of steric hindrance such that the cumulative $^{-}E_s$ value (Taft's steric hindrance constant) is greater than about 1.75 as calculated from the values given for primary amines in Table V from the article by D. F. DeTar, *Journal of Organic Chemistry, 45,* 5174 (1980).

Typical di-tertiaryaminoethers and di-mixed-tertiary- and secondary aminoethers which may be employed in the process of this invention may be represented by one of the following general formulae:

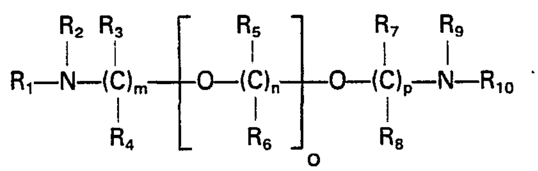

or

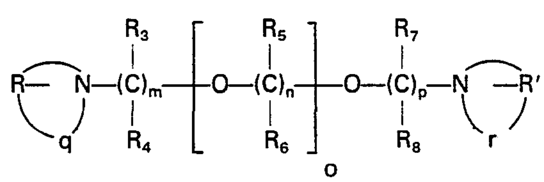

or

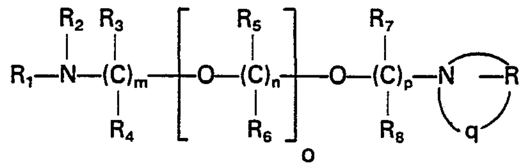

wherein $R_1$, $R_2$, $R_9$ and $R_{10}$ are each independently alkyl, hydroxyalkyl, cycloalkyl or hydroxycycloalkyl radicals, R and R′ which are optional substituents on the nitrogen heterocyclic ring can each be independently hydroxyl, alkyl, hydroxyalkyl, cycloalkyl or hydroxycycloalkyl radicals, $R_3$, $R_4$, $R_5$, $R_6$, $R_7$ and $R_8$ are each independently hydrogen or $C_1$—$C_4$ alkyl or hydroxyalkyl radicals, m, n and p are positive integers each independently ranging from 2 to 4, o is either zero or a positive integer ranging from 1 to 10, and q and r, representing the number of carbon atoms in the heterocyclic ring, are each independently positive integers ranging from 3 to 8. If one of the amino groups is a severely sterically hindered secondary amine, $R_1$ or $R_{10}$, but not both, may be hydrogen, but only if $R_2$ or $R_9$ (whichever is relevant) is an alkyl or hydroxyalkyl radical having 2 to 8 carbon atoms or a cycloalkyl or hydroxycycloalkyl radical having 3 to 8 carbon atoms, with the proviso that if the carbon atom of $R_2$ or $R_9$ directly bonded to the nitrogen atom is secondary, at least one of $R_3$ or $R_4$, or $R_7$ or $R_8$ (whichever is relevant) is an alkyl or hydroxyalkyl radical, and that if the carbon atom of $R_2$ or $R_9$ directly bonded to the nitrogen atom is primary, both $R_3$ and $R_4$, or both $R_7$ and $R_8$ (whichever is relevant) are alkyl or hydroxyalkyl radicals. In the latter case, $R_2$ is preferably a tert-butyl, cycloalkyl, 1 - methyl - 1 - ethylpropyl or tert-amyl radical, most preferably a tert-butyl radical.

Representative di-tertiary and di-mixed aminoethers generally useful for $H_2S$ removal include, for example, 1,2 - bis - (N,N - dimethylaminoethoxy)ethane, bis - (N - pyrrolidinylethyl)ether, N - pyrrolidinylethoxyethyl - N,N - diethylamine, 1,2 - bis - (pyrrolidinylethoxy)ethane, 1,2 - bis - (pyrrolidinylethoxyethoxy)ethane, 1,2 - bis - (3 - pyrrolidinyl - n - propoxy)ethane, 1,2 - bis - (piperidinylethoxy)ethane, 1,3 - bis - (piperidinyl - n - propoxy)propane, 1 - (pyrrolidinylethoxy) - 2 - (t - butylaminoethoxy)ethane, 1,2 - bis - (azepanylethoxy)ethane, 1 - (pyrrolidinylethoxy) - 2 - (piperidinylethoxy)ethane, 1 - (pyrrolidinylethoxy) - 2 - [2 - (t - butylamino)propoxy]ethane, 1 - (pyrrolidinylethoxy) - 2 - (2 - isopropylamino)propoxyethane, 1 - (pyrrolidinylethoxy) - 2 - (1 - methyl - 1 - ethylpropylaminoethoxy)ethane, 1 - (pyrrolidinylethoxy) - 2 - (t - amylaminoethoxy)ethane, and the like.

The preferred ditertiaryaminoethers for general use in the practice of this invention are represented by the following general formula:

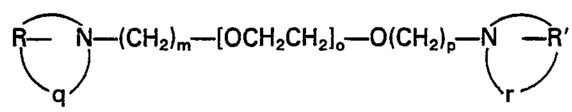

wherein R and R′ are each independently alkyl or cycloalkyl radicals which are substituents on the nitrogen heterocyclic ring, q and r are each independently positive integers ranging from 3 to 5, m and p are positive integers each independently ranging from 2 to 3, and o is either zero or a positive integer ranging from 1 to 4. Of this group the most preferred di-tertiaryaminoether is one or more compounds bis - (N - pyrrolidinylethyl)ether, 1,2 - bis - (pyrrolidinylethoxyethoxy)ethane, 1,2 - bis - (pyrrolidinylethoxy)ethane, 1,2 - bis - (piperidinylethoxy)ethane, 1,2 - bis - (3 - pyrrolidinyl - n - propoxy)ethane and 1 - (pyrrolidinylethoxy) - 2 - (t - butylaminoethoxy)ethane.

The novel classes of di-secondaryaminoethers and di-mixed-secondary-and tertiaryaminoethers which are useful in the $H_2S$ removal process herein may be defined in general by the formula:

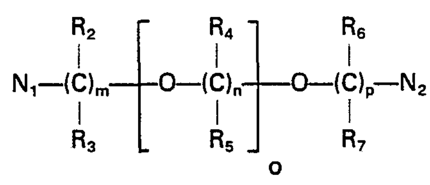

wherein if the diaminoether is a di-secondaryaminoether, $N_1$ and $N_2$ are represented by the formulae:

$$—NH—R_1 \text{ and } —NH—R_8, \text{ respectively,}$$

where $R_1$ and $R_8$ are each independently a primary alkyl radical having 1 to 8 carbon atoms, a primary hydroxyalkyl radical having 2 to 8 carbon atoms, or a secondary or tertiary alkyl or hydroxyalkyl or cycloalkyl or hydroxycycloalkyl radical having 3 to 8 carbon atoms, $R_2$, $R_3$, $R_4$, $R_5$, $R_6$ and $R_7$ are each

independently hydrogen or a $C_1$—$C_4$ alkyl or $C_1$—$C_4$ hydroxyalkyl radical, with the proviso that when the carbon atoms of $R_1$ and $R_8$ directly bonded to the nitrogen atom are primary, $R_2$, $R_3$, $R_6$ and $R_7$ are all alkyl or hydroxyalkyl radicals, and when the carbon atoms of $R_1$ and $R_8$ directly bonded to the nitrogen atom are secondary, at least one of $R_2$ or $R_3$ and at least one of $R_6$ or $R_7$ are alkyl or hydroxyalkyl radicals, m, n and p are positive integers from 1 to 10; and o is either 0 or a positive integer from 1 to 10; and

wherein if the diaminoether is a di-secondary- and tertiaryaminoether, $N_1$ and $N_2$ are represented by the formulae:

—NH—$R_1$ and 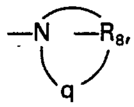, respectively,

where $R_1$ and $R_8$ are each independently a primary alkyl radical having 1 to 8 carbon atoms, a primary hydroxyalkyl radical having 2 to 8 carbon atoms, or a secondary or tertiary alkyl or hydroxyalkyl or cycloalkyl or hydroxycycloalkyl radical having 3 to 8 carbon atoms, and $R_8$, representing one or more optional substituents on the nitrogen heterocyclic ring, may also be a hydroxyl group, q represents the number of carbon atoms in the heterocyclic ring and is a positive integer from 3 to 8, $R_2$, $R_3$, $R_4$, $R_5$, $R_6$, and $R_7$ are each independently hydrogen or a $C_1$—$C_4$ alkyl or $C_1$—$C_4$ hydroxyalkyl radical, with the proviso that when the carbon atom of $R_1$ directly bonded to the nitrogen atom is primary, both $R_2$ and $R_3$ are alkyl or hydroxyalkyl radicals, and when the carbon atom of $R_1$ directly bonded to the nitrogen atom is secondary, at least one of $R_2$ or $R_3$ is an alkyl or hydroxyalkyl radical, m, n and p are positive integers from 2 to 4, and o is either 0 or a positive integer from 1 to 10.

The novel di-secondaryaminoethers useful in the practice of this invention are preferably represented by the following general formula:

$$R_1-NH-\overset{\overset{\displaystyle R_2}{|}}{C}HCH_2-\left[O-CH_2CH_2\right]_o-OCH_2\overset{\overset{\displaystyle R_6}{|}}{C}H-NH-R_8$$

wherein $R_1$ and $R_8$ are each independently secondary alkyl or secondary hydroxyalkyl radicals having 3 to 8 carbon atoms, or tertiary alkyl or tertiary hydroxyalkyl radicals having 4 to 8 carbon atoms, $R_2$ and $R_6$ are each independently hydrogen or $C_1$—$C_4$ alkyl radicals, with the proviso that when $R_1$ and $R_8$ are secondary alkyl radicals, both $R_2$ and $R_6$ are $C_1$—$C_4$ alkyl radicals, and o is either zero or a positive integer ranging from 1 to 4. More preferably, $R_1$ and $R_8$ are tertiary butyl, tertiary amyl or cycloalkyl radicals, e.g., cyclopentyl, cyclohexyl or isopropyl radicals. Most preferably, $R_1$ and $R_8$ are both tertiary butyl radicals and $R_2$ and $R_6$ are hydrogen. When $R_1$ and $R_8$ are secondary alkyl radicals, e.g., isopropyl, it is preferred that each of $R_2$ and $R_6$ is a methyl radical. The symbol o is more preferably zero or an integer of 1 or 2.

The novel di-secondaryaminoethers herein may be prepared by reacting the appropriate primary amine, e.g., $R_1$—$NH_2$ and $R_8$—$NH_2$, with a di-(haloalkoxy)alkane or bis-sulfonate ester of a glycolic ether at elevated temperatures and under conditions such that the haloacid or sulfonic acid is eliminated. For example, 1,2 - bis - (t - butylaminoethoxy)ethane may be prepared by reacting t-butylamine with 1,2 - (bis - chloroethoxy)ethane in a solvent such as ethylene glycol monomethyl ether under reflux conditions. The product can be simply recovered by treating the hot solution with an excess amount of an alkaline hydroxide followed by filtration and distillation of the product.

The preferred class of novel di-mixed secondary- and tertiaryaminoethers herein may be represented by the formula:

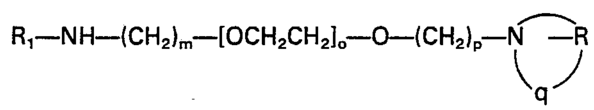

wherein R is an alkyl or cycloalkyl radical which is a substituent on the nitrogen heterocyclic ring, $R_1$ is a branched chain alkyl radical having 3 to 8 carbon atoms of which the carbon atom directly bonded to the nitrogen atom is tertiary, q is a positive integer ranging from 3 to 5, m and p are positive integers each independently ranging from 2 to 3, and o is either zero or a positive integer ranging from 1 to 4.

These novel di-mixed aminoethers may be prepared by a noncatalytic process comprising (1) contacting a heterocycle having 4 to 9 ring atoms with a haloalkoxyalkanol or haloalkoxyalkoxyalkanol at elevated temperatures, preferably in the presence of a solvent, (2) reacting the resultant isolated N - (hydroxyalkoxyalkyl) - heterocycle or N - (hydroxyalkoxyalkoxyalkyl)heterocycle from step (1) with a thionyl halide such as thionyl chloride, preferably in a solvent, to obtain an N - (haloalkoxyalkyl)heterocycle or N - (haloalkoxyalkoxyalkyl)heterocycle, and (3) reacting the isolated product of step (2) with a primary alkyl amine at elevated temperatures, preferably in the presence of a solvent, to obtain the di-mixed aminoether.

Typical primary alkylamines useful in the above preparation process include isopropylamine, tertiarybutylamine and tert-amylamine. Typical haloalkoxyalkoxyalkanols include, e.g., 2-chloroethoxyethoxyethanol. Suitable heterocycles include azepane, pyrrolidine, piperidine, and the like. Typical solvents for steps (1) and (3) include lower alkanols and ethers such as, e.g., methanol, ethanol, propanol, ethylene glycol monomethyl ether, diethylether, tetrahydrofuran, and the like. Preferably, the solvent is a polar solvent and most preferably is ethanol or ethylene glycol monomethyl ether. The solvents generally used for step (2) are non-polar solvents such as, e.g., toluene.

Steps (1) and (3) of the above preparation process are preferably carried out at temperatures ranging from 50°C to about 250°C, more preferably from 100°C to about 200°C. Step (3) is preferably carried out in an autoclave. During step (2) the N - (hydroxyalkoxyalkoxyalkyl) heterocycle is generally added as a solution dropwise to the thionyl halide in solution, with no elevated temperatures ordinarily employed.

Representative novel diaminoethers herein from all categories include, for example, bis - (tertiarybutylaminoethyl)ether, 1,2 - bis - (tertiarybutylaminoethoxy)ethane, 1,2 - bis - (tertiarybutylaminoethoxyethoxy)ethane, bis - [2 - (isopropylamino)propyl]ether, 1,2 - [2 - (isopropylamino) - propoxy]ethane, 1 - (pyrrolidinylethoxy) - 2 - t - butylaminoethoxy)ethane, 1 - (pyrrolidinylethoxy) - 2 - [2 - (t - butylamino) - propoxy]ethane, 1 - (pyrrolidinylethoxy) - 2 - [2 - (2 - isopropylamino)propoxy]ethane, 1 - (pyrrolidinylethoxy) - 2 - (1 - methyl - 1 - ethylpropylaminoethoxy)ethane, 1 - (pyrrolidinylethoxy) - 2 - (t - amylaminoethoxy)ethane, 1 - (piperidinyl - n - propoxy) - 2 - (t - butylaminoethoxy)ethane, and 1 - (azepanylethoxy) - 2 - (t - butylaminoethoxy)ethane.

The most preferred novel diaminoethers of this invention from each category are 1,2 - bis - (tertiarybutylaminoethoxy)ethane and 1 - (pyrrolidinylethoxy) - 2 - (t - butylaminoethoxy)ethane.

The diaminoethers used in the selective absorption process of the present invention usually have a $pK_a$ value at 20°C greater than 8.6, preferably greater than about 9.5 and more preferably the $pK_a$ value of the diaminoether will range between about 9.5 and about 10.6. If the $pK_a$ is less than 8.6 the reaction with $H_2S$ is decreased, whereas if the $pK_a$ of the diaminoether is much greater than about 10.6 an excessive amount of steam is required to regenerate the solution. Also, to ensure operational efficiency with minimal losses of the diaminoether, the diaminoether may have a relatively low volatility. For example, the boiling point of the amine (at 760 mm) may be typically greater than about 180°C, preferably greater than 200°C, and more preferably greater than 225°C.

The diaminoethers herein may also be characterized by their high solubility in water at selective $H_2S$ removal conditions, and most of the compounds are also generally soluble in polar organic solvent systems which may or may not contain water. The term "absorbent solution" as used herein refers to solutions wherein the diaminoether is dissolved in a solvent, preferably selected from water or a physical absorbent or a mixture of water and physical absorbent. Solvents which are physical absorbents (as opposed to the diaminoethers which are chemical absorbents) by definition function to solubilize the diaminoethers and include, e.g., aliphatic acid amides, N-alkylated pyrrolidones, sulfones, sulfoxides, glycols and the mono- and diethers thereof. The preferred physical absorbents herein are sulfones, and most particularly, sulfolane.

The absorbent solution may have a concentration of diaminoether of about 0.1 to 6 moles per liter of the total solution, and preferably 1 to 4 moles per liter, depending primarily on the specific diaminoether employed and the solvent system utilized. If the solvent system is a mixture of water and a physical absorbent, the typical effective amount of the physical absorbent employed may vary from 0.1 to 5 moles per liter of total solution, and preferably from 0.5 to 3 moles per liter, depending mainly on the type of diaminoether being utilized. The dependence of the concentration of diaminoether on the particular compound employed is significant because increasing the concentration of diaminoether may reduce the basicity of the absorbent solution, thereby adversely affecting its selectivity for $H_2S$ removal, particularly if the diaminoether has a specific aqueous solubility limit which will determine maximum concentration levels within the range given above. It is important, therefore, that the proper concentration level appropriate for each particular diaminoether be maintained to insure satisfactory results.

The solution of this invention may include a variety of additives typically employed in selective gas removal processes, e.g., antifoaming agents, antioxidants, corrosion inhibitors, and the like. The amount of these additives will typically be in the range that they are effective, i.e., an effective amount.

Also, the diaminoethers described herein may be admixed with other amino compounds (i.e., any amines other than those employed as diaminoethers herein) as a blend, preferably with methyldiethanolamine, to form the absorbent solution. The ratio of the respective amino compounds may vary widely, for example, from 1 to 99 weight percent of the diaminoethers herein.

Three characteristics which are of ultimate importance in determining the effectiveness of the diaminoethers herein for $H_2S$ removal are "selectivity", "loading" and "capacity". The term "selectivity" as used throughout the specification is defined as the following mole ratio fraction:

$$\frac{(\text{moles of } H_2S/\text{moles of } CO_2) \text{ in liquid phase}}{(\text{moles of } H_2S/\text{moles of } CO_2) \text{ in gaseous phase}}$$

The higher this fraction, the greater the selectivity of the absorbent solution for the $H_2S$ in the gas mixture.

By the term "loading" is meant the concentration of the $H_2S$ and $CO_2$ gases physically dissolved and chemically combined in the absorbent solution as expressed in moles of gas per moles of the amine. The best diaminoethers are those which exhibit good selectivity up to a relatively high loading level. The diaminoethers used in the practice of the present invention may have a "selectivity" of not substantially less than 10 at a "loading" of 0.1 moles, preferably, a "selectivity" of not substantially less than 10 at a loading of 0.2 or more moles of $H_2S$ and $CO_2$ per mole of the diaminoether.

"Capacity" is defined as the moles of $H_2S$ loaded in the absorbent solution at the end of the absorption step minus the moles of $H_2S$ loaded in the absorbent solution at the end of the desorption step. High capacity enables one to reduce the amount of amine solution to be circulated and use less heat or steam during regeneration.

The diaminoethers herein are typically capable to reducing the $H_2S$ in gaseous mixtures to a relatively low level, e.g., less than about 200 ppm, and typically have a relatively high capacity for $H_2S$, e.g., greater than about 0.2 mole of $H_2S$ per mole of amine. The diaminoethers may be characterized as having a "kinetic selectivity" for $H_2S$, i.e., a faster reaction rate for $H_2S$ than for $CO_2$ at absorption conditions. In addition, they may be characterized as having a higher capacity for $H_2S$ at equivalent kinetic selectivity for $H_2S$ over $CO_2$. This higher capacity results in the economic advantage of lower steam requirements during regeneration.

The normally gaseous stream herein necessarily includes $H_2S$, ordinarily in gaseous form. (The term "normally" refers to ambient conditions of temperature and pressure). It may optionally include other gases such as $CO_2$, $N_2$, $CH_4$, $H_2$, CO, $H_2O$, COS, HCN, $C_2H_4$, $NH_3$, and the like. Often such gas mixtures are found in combustion gases, refinery gases, town gas, natural gas, syn gas, water gas, propane, propylene, heavy hydrocarbon gases, etc. The absorbent solution herein is particularly effective when the gaseous mixture is a gas, obtained, for example, from shale oil retort gas, coal or gasification of heavy oil with air/steam or oxygen/steam, thermal conversion of heavy residual oil to lower molecular weight liquids and gases, or in sulfur plant tail gas clean-up operations.

The absorption step of this invention generally involves contacting the normally gaseous stream with the absorbent solution in any suitable contacting vessel. In such processes, the normally gaseous mixture from which the $H_2S$ is to be removed may be brought into intimate contact with the absorbent solution using conventional means, such as a tower of vessel packed with, for example, rings or with sieve plates, or a bubble reactor.

In a typical mode of practising the invention, wherein $H_2S$ is selectively absorbed from a mixture also containing $CO_2$, the absorption step is conducted by feeding the normally gaseous mixture into the lower portion of the absorption tower while fresh absorbent solution is fed into the upper region of the tower. The gaseous mixture, freed largely from the $H_2S$, emerges from the upper portion of the tower, and the loaded absorbent solution, which contains the selectively absorbed $H_2S$, leaves the tower near or at its bottom. Preferably, the inlet temperature of the absorbent solution during the absorption step is in the range of from about 20 to about 100°C, and more preferably from 40 to about 60°C. Pressures may vary widely; acceptable pressures are between 0.4 and 140.6 kg/cm$^2$, preferably 1.4 and 105.5 kg/cm$^2$, and most preferably 1.8 and 70.3 kg/cm$^2$ in the absorber. The contacting takes place under conditions such that the $H_2S$ is selectively absorbed by the solution.

The absorption conditions and apparatus are designed so as to minimize the residence time of the liquid in the absorber to reduce $CO_2$ pickup while at the same time maintaining sufficient residence time of gas mixture with liquid to absorb a maximum amount of the $H_2S$ gas. The amount of liquid required to be circulated to obtain a given degree of $H_2S$ removal will depend on the chemical structure and basicity of the diaminoether and on the partial pressure of $H_2S$ in the feed gas. Gas mixtures with low partial pressures such as those encountered in thermal conversion processes will require more liquid under the same absorption conditions than gases with higher partial pressures such as shale oil retort gases.

A typical procedure for the selective $H_2S$ removal phase of the process comprises selectively absorbing $H_2S$ via countercurrent contact of the gaseous mixture containing $H_2S$ and $CO_2$ with the solution of the diaminoether in a column containing a plurality of trays at a low temperature, e.g., below 45°C, and at a gas velocity of at least about 9.1 cm/sec (based on "active" or aerated tray surface), depending on the operating pressure of the gas, said tray column having fewer than 20 contacting trays, with, e.g., 4—16 trays being typically employed. Alternatively, packed beds may be used in place of trays.

After contacting the normally gaseous mixture with the absorbent solution, which becomes saturated or partially saturated with $H_2S$, the solution may be at least partially regenerated so that it may be recycled back to the absorber. As with absorption, the regeneration may take place in a single liquid phase. Regeneration or desorption of the acid gases from the absorbent solution may be accomplished by conventional means such as pressure reduction of the solution or increase of temperature to a point at which the absorbed $H_2S$ flashes off, or by passing the solution into a vessel of similar construction to that used in the absorption step, at the upper portion of the vessel, and passing an inert gas such as air or nitrogen or preferably steam upwardly through the vessel. The temperature of the solution during the regeneration step should be in the range from about 50 to about 170°C, and preferably from about 80 to 120°C, and the pressure of the solution on regeneration should range from about 0.04 to about 7.0 kg/cm$^2$,

preferably 0.07 to about 3.5 kg/cm². The absorbent solution, after being cleansed of at least a portion of the $H_2S$ gas, may be recycled back to the absorbing vessel. Makeup absorbent may be added as needed.

In the preferred regeneration technique, the $H_2S$-rich solution is sent to the regenerator wherein the absorbed components are stripped by the steam which is generated by reboiling the solution. Pressure in the flash drum and stripper is usually 0.07 to about 3.5 kg/cm², preferably 1.05 to about 2.1 kg/cm², and the temperature is typically in the range from about 50 to 170°C, preferably 80 to 120°C. Stripper and flash temperatures will, of course, depend on stripper pressure, thus, at about 1.05 to 2.1 kg/cm² stripper pressures, the temperature will be about 80 to 120°C during desorption. Heating of the solution to be regenerated may very suitably be effected by means of indirect heating with low-pressure steam. It is also possible, however, to use direct injection of steam.

In one embodiment for practising the entire process herein, as illustrated in Figure 1, the gas mixture to be purified is introduced through line *1* into the lower portion of a gas-liquid countercurrent contacting column *2*, said contacting column having a lower section *3* and an upper section *4*. The upper and lower sections may be segregated by one or a plurality of packed beds as desired. The absorbent solution as described above is introduced into the upper portion of the column through a pipe *5*. The solution flowing to the bottom of the column encounters the gas flowing countercurrently and dissolves the $H_2S$ preferentially. The gas freed from most of the $H_2S$ leaves through a pipe *6* for final use. The solution, containing mainly $H_2S$ and some $CO_2$, flows toward the bottom portion of the column from which it is discharged through pipe *7*. The solution is then pumped via optional pump *8* through an optional heat exchanger and cooler *9* disposed in pipe *7*, which allows the hot solution from the regenerator *12* to exchange heat with the cooler solution from the absorber column *2* for energy conservation. The solution is entered via pipe *7* to a flash drum *10* equipped with a line (not shown) which vents to line *13* and then introduced by pipe *11* into the upper portion of the regenerator *12*, which is equipped with several plates and effects the desorption of the $H_2S$ and $CO_2$ gases carried along in the solution. This acid gas mixture is passed through a pipe *13* into a condenser *14* wherein cooling and condensation of water and amine solution from the gas occur. The gas then enters a separator *15* where further condensation is effected. The condensed solution is returned through pipe *16* to the upper portion of the regenerator *12*. The gas remaining from the condensation, which contains $H_2S$ and some $CO_2$, is removed through pipe *17* for final disposal (e.g., to a vent or incinerator or an apparatus which converts the $H_2S$ to sulfur, such as a Claus unit or a Stretford conversion unit (not shown)).

The solution is liberated from most of the gas which it contains while flowing downward through the regenerator *12* and leaves through pipe *18* at the bottom of the regenerator for transfer to a reboiler *19*. Reboiler *19*, equipped with an external source of heat (e.g., steam is injected through pipe *20* and the condensate leaves through a second pipe (not shown), vaporizes a portion of this solution (mainly water) to drive further $H_2S$ therefrom. The $H_2S$ and steam driven off are returned via pipe *21* to the lower section of the regenerator *12* and exited through pipe *13* for entry into the condensation stages of gas treatment. The solution remaining in the reboiler *19* is drawn through pipe *22*, cooled in heat exchanger *9*, and is introduced via the action of pump *23* (optional if pressure is sufficiently high) through pipe *5* into the absorber column *2*.

The diaminoethers herein are found to be superior to those used in the past, particularly to MDEA and DEAE, in terms of both selectivity and capacity for maintaining selectivity over a broad loading range. Typically, a gaseous stream to be treated having a 1:10 mole ratio of $H_2S$:$CO_2$ from an apparatus for thermal conversion of heavy residual oil, or a Lurgi coal gas having a mole ratio of $H_2S$:$CO_2$ of less than 1:10 will yield an acid gas having a mole ratio of $H_2S$:$CO_2$ of about 1:1 after treatment by the process of the present invention. The process herein may be used in conjunction with another $H_2S$ selective removal process; however, it is preferred to carry out the process of this invention by itself, since the diaminoethers are extremely effective by themselves in preferential absorption of $H_2S$.

The invention is illustrated further by the following examples, which, however, are not to be taken as limiting in any respect. All parts and percentages, unless expressly stated to be otherwise, are by weight. All $pK_a$ values, unless otherwise noted, are measured at 20°C. The abbreviation b.p. means boiling point.

Examples of di-tertiaryaminoethers

Example 1

Preparation of 1,2-bis-(pyrrolidinylethoxy)ethane (BIS-1)

To a solution of 153 g of pyrrolidine in 950 ml of tetrahydrofuran (THF) was added 217.8 g of triethylene glycol ditosylate in 1400 ml of THF at ambient temperature over a period of 2.5 hours. The reaction was refluxed overnight. The solvent was then removed at reduced pressure and the residue extracted several times with diethyl ether. Evaporation of the ether and distillation of the residue resulted in an 85% yield of the product as a colorless oil with a b.p. of 123—5°C at 0.15 mm.

Analysis calculated for $C_{14}H_{28}N_2O_2$: %C, 65.62; %H, 10.94; %N, 10.94. Found: %C, 65.43; %H, 11.15; %N, 10.79.

Example 2

Alternative preparation of 1,2-bis-(pyrrolidinylethoxy)ethane (BIS-1)

To a solution of 500 g (7.05 moles) of pyrrolidine in 1600 ml of ethylene glycol monomethyl ether was

added 298 g (1.6 moles) of 1,2-bis-(chloroethoxy)ethane in 250 ml of ethylene glycol monomethyl ether. The reaction mixture was refluxed overnight, cooled slightly, and reacted with 197 g (3.52 moles) of KOH. After heating for one hour the reaction mixture was cooled, filtered, and the solvent was stripped on a rotary evaporator at reduced pressure. Extraction of the residue with ether and distillation of the ether residue yielded 682 g (83%) of the bis-aminoether (b.p.=108—110°C at 0.06 mm).

Example 3
Preparation of bis-(pyrrolidinylethoxyethyl)ether (BIS-2)

The experimental procedure described in Example 1 was repeated using pyrrolidine and tetraethylene glycol ditosylate to yield the desired product, which had a b.p. of 150°C at 0.25 mm.

Analysis calculated for $C_{16}$ $H_{32}$ $N_2O_3$: %N, 9.33. Found: %N, 8.83.

Example 4
Preparation of 1,2-bis-(3-pyrrolidinyl-n-propoxy)ethane (BIS-P)

To a suspension of 25 g of $LiAlH_4$ in 500 ml of THF was added 140 g of dimethyl-4,7-dioxadecanedioate in 200 ml of THF at a rate sufficient to maintain reflux.

Reflux was continued for an additional 5 hours. The cooled reaction mixture was then decomposed sequentially with 25 g water, 25 g 15% NaOH and 64 ml of water, filtered, and distilled to give a 76% yield of 1,2 - bis - (3 - hydroxy - n - propoxy)ethane, a glycolic ether which had a b.p. of 132°C at 0.3 mm.

To 63 g of the above glycolic ether in 64 ml of dry pyridine cooled to −10°C was added 135.6 g of p-toluenesulfonyl chloride in 200 ml of dry pyridine over a period of 0.75 hr. The reaction mixture was maintained at −10°C for an additional two hours and then was poured into one liter of ice water. The precipitated product, 1,2 - bis - (3 - hydroxy - n - propoxy)ethane ditosylate, was filtered, washed with water, and dried to give an 83% yield, m.p. 59—60°C.

A total of 30 g pyrrolidine was added to a warm solution of 39 g of the 1,2 - bis - (3 - hydroxy - n - propoxy)ethane ditosylate in 200 ml of ethylene glycol monomethyl ether and refluxed overnight. Ten grams of KOH was added and reflux was continued for 0.5 hour. The cooled reaction mixture was filtered and the filtrate concentrated under reduced pressure. The residue was stirred with 300 ml of THF, filtered, and distilled. The product was obtained in 83% yield and had a b.p. of 130°C at 0.1 mm.

Analysis calculated for $C_{16}$ $H_{32}$ $N_2O_2$: %N, 9.86. Found: %N, 9.80.

Example 5
Selective $H_2S$ removal from a mixture containing $H_2S$ and $CO_2$

Fig. 2 illustrates the sparged absorber unit, operated on a semi-batch mode, used to evaluate the selectivity for $H_2S$ removal of the diaminoethers of the invention herein. A gas mixture comprised of 10% $CO_2$, 1% $H_2S$ and 89% $N_2$, expressed in volume percent, respectively, was passed from a gas cylinder (not shown) through line *30* to a meter *31* measuring the rate at which the gas is fed to the absorber. For all examples this rate was 3.6 liters per minute. The gas was then passed through line *32* to a gas chromatography column (not shown) continuously monitoring the composition of the inlet gas and through lines *33* and *34* to a sparged absorber unit *35*, which is a cylindrical glass tube 45 cm high and 3.1 cm in diameter charged with 100 ml of the absorbent amine solution *36*. The gas was passed through the solution at a solution temperature of 40°C, and 10-ml samples of the solution were periodically removed from the bottom of the absorber unit through lines *34* and *37* to be analyzed for $H_2S$ and $CO_2$ content. The $H_2S$ content in the liquid sample was determined by titration with silver nitrate. The $CO_2$ content of the liquid sample was then analyzed by acidifying the sample with an aqueous solution of 10% HCl and measuring the evolved $CO_2$ by weight gain on NaOH-coated asbestos.

While the solution was being periodically withdrawn from the bottom of the absorber unit, the gas mixture was removed from the top thereof via line *38* to a trap *39* which served to scrub out any $H_2S$ in the outlet gas. The resulting gas could optionally then be passed via lines *40* and *41* for final disposal or via line *42* to a gas chromatography column (not shown) for periodic evaluation of the composition of the outlet gas to check for system leaks. For purposes of the examples, the contents of $H_2S$ and $CO_2$ in the inlet phases were measured and the contents of $H_2S$ and $CO_2$ in the liquid phase were determined as described above. These data were used to calculate selectivity values of the amine as defined above, which were plotted as a function of the loading of the absorbent solution with $H_2S$ and $CO_2$, in units of moles acid gas per moles of the diaminoether.

The above procedure was employed using as the absorbent solution aqueous 1.5 M solutions of 1,2 - bis - (pyrrolidinylethoxy)ethane (BIS-1) ($pK_a$=9.55) and 1,2 - bis - (piperidinylethoxy)ethane (BIS-PIP) ($pK_a$=9.61, b.p.=135°C at 0.09 mm), and an aqueous 3M solution of methyldiethanolamine (MDEA) as comparison (representing an amine often used for selective $H_2S$ removal). The selectivity plots for all three amines are shown in Fig. 3.

Example 6

The procedure of Example 5 was repeated using as the absorbent solution aqueous 1.5 M solutions of bis - (N - pyrrolidinylethyl)ether (BIS-0) ($pK_a$=9.75; b.p.=96°C at 0.37 mm), 1,2 - bis - (3 - pyrrolidinyl - n - propoxy)ethane (BIS-P) ($pK_a$=10.23, b.p.=130°C at 0.1 mm), and 1,2 - bis - (pyrrolidinylethoxy-

ethoxy)ethane (BIS-2) ($pK_a$=9.63; b.p.=150°C at 0.25 mm). A selectivity plot for all three amines was recorded as given in Fig. 4.

Examples of di-mixed aminoether

Example 7

Preparation of 1-(pyrrolidinylethoxy)-2-(t-butylaminoethoxy)ethane (PETBEE)

A total of 295 g of pyrrolidine and 250 g of 2-chloroethoxyethoxyethanol in 750 ml of ethanol was refluxed for 5 hours. The reaction mixture was then treated with 106 g of KOH and refluxed for an additional hour. Filtration and distillation yielded 229 g of N-(hydroxyethoxyethoxyethyl) pyrrolidine (b.p.=145°C at 3 mm).

A solution of 26.4 g of the above-mentioned amino alcohol in 150 ml of toluene was added, dropwise, to 16.6 g of thionyl chloride in 200 ml of toluene. One hour after addition was complete the reaction mixture had separated into two liquid phases. The lower phase was withdrawn, treated with a saturated aqueous sodium carbonate solution and extracted with ether. Distillation of the dried ether solution yielded 14 g of N - (chloroethoxyethoxyethyl)pyrrolidine (b.p.=120°C at 3 mm).

A solution of 54.8 g of the pyrrolidine derivative thus prepared and 37.2 g of tertiary butylamine in 60 ml of methanol was heated for 3 hours at 150°C in an autoclave. The cooled reaction mixture was removed from the autoclave and treated with 15.6 g of KOH. Filtration and distillation produced 1 - (pyrrolidinylethoxy) - 2 - (tert - butylaminoethoxy)ethane in a 53% yield (b.p.=104°C at 0.11 mm).

Example 8

Selective $H_2S$ removal from a mixture containing $H_2S$ and $CO_2$

The procedure of Example 5 was repeated using as the absorbent solution an aqueous 1.5 M solution of 1 - (pyrrolidinylethoxy) - 2 - (tert - butylaminoethoxy)ethane (PETBEE) ($pK_a$=9.25). As comparisons, aqueous 3 M solutions of t-butylaminoethoxyethanol) (TBEE) and methyldiethanolamine (MDEA) were employed using the same conditions, and a selectivity plot for each amine was recorded as given in Fig. 5.

The results shown in Fig. 5 indicated that the di-mixed aminoether of the present invention is superior to the monoaminoether alcohol (TBEE) and the tertiary monoamine (MDEA).

Examples of di-secondaryaminoether

Example 9

Preparation of 1,2-bis-(t-butylaminoethoxy)ethane (BIS-TB)

A solution of 2.5 moles of t-butylamine and 0.5 mole of 1,2 - (bis - chloroethoxy)ethane in 600 ml of ethylene glycol monomethyl ether was refluxed for three days. Potassium hydroxide, in an amount of 1.2 moles, was added, portionwise, to the hot solution and refluxing was continued for one hour. The cooled reaction was filtered, the solvent stripped, and the residue distilled. The recovered distillate had a b.p. of 104—107°C (0.25 mm), and the yield was 75%.

The product characterized by $^1H$ nmr showed signals at 1.1 (s, 18 H); 2.74 (t, 4 H); 3.6 (t, 8H). Analysis calculated for $C_{14}H_{32}N_2O_2$: %N, 10.77. Found: %N, 10.34.

Example 10

Selective $H_2S$ removal from a mixture containing $H_2S$ and $CO_2$

The procedure of Example 5 was repeated using as the absorbent solution aqueous 1.5 M solutions of 1,2 - bis - (tertiarybutylaminoethoxy)ethane (BIS-TB) and 1,2 - bis - (isopropylaminoethoxy)ethane (BIS-IP) (control) and aqueous 3M solutions of t-butylaminoethoxyethanol (TBEE) (comparison) and methyldiethanolamine (MDEA) (control) using the same conditions. The selectivity data for each amine were plotted on the graph as shown in Fig. 6.

The results shown in Fig. 6 indicate that the di-severely sterically hindered secondary aminoethers of the present invention are far superior to the di-secondary aminoether that is not severely sterically hindered (BIS-IP), the monoamino severely sterically hindered aminoether alcohol (TBEE), and the tertiary amine (MDEA).

The data in Figs. 3, 5 and 6 also show that the diaminoethers of the present invention have very high capacity for both $H_2S$ and $CO_2$ compared to methyldiethanolamine (MDEA) in addition to high $H_2S$ selectivities. It will be apparent from an inspection of the data in Figs. 3, 5 and 6 that if the absorption process is conducted under conditions such that the diaminoether has a long contact time with the gases to be absorbed, the selectivity for $H_2S$ decreases, but the overall capacity for both $CO_2$ and $H_2S$ remains rather high. Therefore, one may, in some instances, wish to carry out a "non-selective" acid gas absorption process to take advantage of the large absorption capacity of the diaminoethers of the invention. Such "non-selective" processes are particularly useful in scrubbing natural gases which contain relatively high levels of $H_2S$ and low to nil levels of $CO_2$. As such, the diaminoethers of the invention may replace some or all of monoethanolamine (MEA) or diethanolamine (DEA) commonly used for such scrubbing processes.

While all examples herein illustrate the superior performance of the diaminoethers for selective $H_2S$ removal using an absorber unit as represented by Fig. 2, it will also be possible to achieve effective selective $H_2S$ removal by using the diaminoethers in an absorption-regeneration unit as depicted in Fig. 1.

In summary, this invention is seen to provide a process for removing $H_2S$ from gaseous streams

utilizing a special class of amino compounds characterized as diaminoethers having tertiary and/or secondary amino groups which have a high selectivity for $H_2S$ in preference to $CO_2$ which selectivity is maintained at high $H_2S$ and $CO_2$ loading levels. This invention also provides a novel class of diaminoethers useful in the process herein.

**Claims**

1. A process for removing $H_2S$ from a normally gaseous stream with an absorbent solution comprising a solvent and a diaminoether wherein both amino groups either are sterically hindered secondary amino groups or are tertiary amino groups or wherein one amino group is a sterically hindered secondary amino group and the other amino group is a tertiary amino group, the degree of steric hindrance being such that the cumulative $^{-}E_s$ value (Taft's steric hindrance constant) is greater than 1.75.

2. A process according to claim 1 wherein the normally gaseous stream also contains $CO_2$, the $H_2S$ is selectively absorbed from the stream and the diaminoether has a $pK_a$ value at 20°C greater than 8.6.

3. A process according to claim 1 or 2 wherein the total concentration of the diaminoether in the absorbent solution is in the range of from about 0.1 to 6 moles per litre, the process is conducted at a temperature ranging from about 20 to 100°C and at a pressure ranging from 0.4 to 140.6 kg/cm$^2$, the absorbent solution is regenerated by heating at a temperature ranging from 50 to about 170°C and at a pressure ranging from 0.07 to about 3.5 kg/cm$^2$ and the solution is stripped of its absorbed $H_2S$.

4. A process according to any one of claims 1 to 3 wherein the diaminoether is of the following formula:

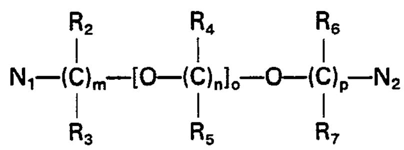

wherein if the diaminoether is a di-tertiaryaminoether, $N_1$ and $N_2$ are represented by the formulae:

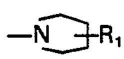 and 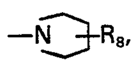, respectively,

wherein $R_1$ and $R_8$ are each independently hydrogen or a hydroxyl, alkyl, hydroxyalkyl, cycloalkyl, hydroxycycloalkyl, alkoxy or hydroxyalkoxy radical, $R_2$, $R_3$, $R_4$, $R_5$, $R_6$ and $R_7$ are each independently hydrogen or a $C_1$ or $C_2$ alkyl or $C_1$ or $C_2$ hydroxyalkyl radical, m, n and p are positive integers from 2 to 5, and o is a positive integer from 1 to 4;
wherein if the diaminoether is a di-secondaryaminoether, $N_1$ and $N_2$ are represented by the formulae:

$$-NH-R_1 \text{ and } -NH-R_8, \text{ respectively,}$$

where $R_1$ and $R_8$ are each independently a primary alkyl radical having 1 to 8 carbon atoms, a primary hydroxyalkyl radical having 2 to 8 carbon atoms, or a secondary or tertiary alkyl or hydroxyalkyl or cycloalkyl or hydroxycycloalkyl radical having 3 to 8 carbon atoms, $R_2$, $R_3$, $R_4$, $R_5$, $R_6$ and $R_7$ are each independently hydrogen or a $C_1$—$C_4$ alkyl or $C_1$—$C_4$ hydroxyalkyl radical, with the proviso that when the carbon atoms of $R_1$ and $R_8$ directly bonded to the nitrogen atom are primary, $R_2$, $R_3$, $R_6$ and $R_7$ are all alkyl or hydroxyalkyl radicals, and when the carbon atoms of $R_1$ and $R_8$ directly bonded to the nitrogen atom are secondary, at least one of $R_2$ or $R_3$ and at least one of $R_6$ or $R_7$ are alkyl or hydroxyalkyl radicals, m, n and p are positive integers from 2 to 4, and o is either 0 or a positive integer from 1 to 10; and
wherein if the diaminoether is a di-secondary- and tertiaryaminoether, $N_1$ and $N_2$ are represented by the formulae:

$-NH-R_1$ and 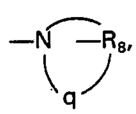, respectively,

where $R_1$ and $R_8$ are each independently a primary alkyl radical having 1 to 8 carbon atoms, a primary hydroxyalkyl radical having 2 to 8 carbon atoms, or a secondary or tertiary alkyl or hydroxyalkyl or cycloalkyl or hydroxycycloalkyl radical having 3 to 8 carbon atoms, and $R_8$, representing one or more optional substituents on the nitrogen heterocyclic ring, may also be a hydroxyl group, q represents the number of carbon atoms in the heterocyclic ring and is a positive integer from 3 to 8, $R_2$, $R_3$, $R_4$, $R_5$, $R_6$ and $R_7$ are each independently hydrogen or a $C_1$—$C_4$ alkyl or $C_1$—$C_4$ hydroxyalkyl radical, with the proviso that when the carbon atom of $R_1$ directly bonded to the nitrogen atom is primary, both $R_2$ and $R_3$ are alkyl or hydroxyalkyl radicals, and when the carbon atom of $R_1$ directly bonded to the nitrogen atom is secondary, at least one of $R_2$ or $R_3$ is an alkyl or hydroxyalkyl radical, m, n and p are positive integers from 2 to 4, and o is either 0 or a positive integer from 1 to 10.

5. A process according to any one of claims 1—4 wherein the diaminoether is 1,2 - bis - (3 - pyrrolidinyl - n - propoxy)ethane, 1,2 - bis - (tertiarybutylaminoethoxy)ethane or 1 - (pyrrolidinylethoxy) - 2 - (t - butylaminoethoxy)ethane.

6. An absorbent solution useful for the process according to any one of the preceding claims which comprises a solvent and a diaminoether wherein both amino groups either are sterically hindered secondary amino groups or wherein one amino group is a sterically hindered secondary amino group and the other amino group is a tertiary amino group, the degree of steric hindrance being such that the cumulative $^{-}E_s$ value (Taft's steric hindrance constant) is greater than 1.75.

7. A solution according to claim 6 wherein the solvent is a physical absorbent.

8. A diaminoether composition having the following formula:

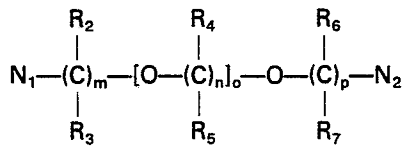

wherein if the diaminoether is a di-secondaryaminoether, $N_1$ and $N_2$ are represented by the formulae:

$$—NH—R_1 \text{ and } —NH—R_8, \text{ respectively,}$$

where $R_1$ and $R_8$ are each independently a primary alkyl radical having 1 to 8 carbon atoms, a primary hydroxyalkyl radical having 2 to 8 carbon atoms, or a secondary or tertiary alkyl or hydroxyalkyl or cycloalkyl, or hydroxycycloalkyl radical having 3 to 8 carbon atoms, $R_2$, $R_3$, $R_4$, $R_5$, $R_6$ and $R_7$ are each independently hydrogen or a $C_1$—$C_4$ alkyl or $C_1$—$C_4$ hydroxyalkyl radical, with the proviso that when the carbon atoms of $R_1$ and $R_8$ directly bonded to the nitrogen atom are primary, $R_2$, $R_3$, $R_6$ and $R_7$ are all alkyl or $C_1$—$C_4$ hydroxyalkyl radicals, and when the carbon atoms of $R_1$ and $R_8$ directly bonded to the nitrogen atom are secondary, at least one of $R_2$ or $R_3$ and at least one of $R_6$ or $R_7$ are alkyl or hydroxyalkyl radicals, m, n and p are positive integers from 2 to 4, and o is either 0 or a positive integer from 1 to 10; and wherein if the diaminoether is a di-secondary- and tertiaryaminoether, $N_1$ and $N_2$ are represented by the formulae:

—NH —$R_1$ and 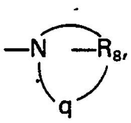, respectively,

where $R_1$ and $R_8$ are each independently a primary alkyl radical having 1 to 8 carbon atoms, a primary hydroxyalkyl radical having 2 to 8 carbon atoms, or a secondary or tertiary alkyl or hydroxyalkyl or cycloalkyl or hydroxycycloalkyl radical having 3 to 8 carbon atoms, and $R_8$, representing one or more optional substituents on the nitrogen heterocyclic ring, may also be a hydroxyl group, q represents the number of carbon atoms in the heterocyclic ring and is a positive integer from 3 to 8, $R_2$, $R_3$, $R_4$, $R_5$, $R_6$ and $R_7$ are each independently hydrogen or a $C_1$—$C_4$ alkyl or $C_1$—$C_4$ hydroxyalkyl radical, with the proviso that when the carbon atom of $R_1$ directly bonded to the nitrogen atom is primary, both $R_2$ and $R_3$ are alkyl or hydroxyalkyl radicals, and when the carbon atom of $R_1$ directly bonded to the nitrogen atom is secondary, at least one of $R_2$ or $R_3$ is an alkyl or hydroxyalkyl radical, m, n and p are positive integers from 2 to 4, and o is either 0 or a positive integer from 1 to 10.

9. A diaminoether according to claim 8 which is 1,2 - bis - (tertiarybutylaminoethoxy)ethane or 1 - (pyrrolidinylethoxy) - 2 - (t - butylaminoethoxy)ethane.

10. A process for preparing the di-secondary aminoether according to claim 8 which comprises reacting a primary amine with a di-(haloalkoxy) alkane or a bis-sulfonate ester of a glycolic ether at an elevated temperature.

11. A process for preparing the di-secondary- and tertiaryaminoether according to claim 8 which comprises:

(a) contacting a heterocyclic compound having 4 to 9 ring atoms with a haloalkoxyalkanol or a haloalkoxyalkoxyalkanol at an elevated temperature;

(b) reacting the product of step (a) with a thionyl halide; and

(c) reacting the product of step (b) with a primary alkylamine at an elevated temperature.

**Patentansprüche**

1. Verfahren zur Entfernung von $H_2S$ aus einem normalerweise gasförmigen Strom mit einer Absorptionslösung, die ein Lösungsmittel und einen Diaminoether enthält, bei dem beide Aminogruppen entweder sterisch gehinderte sekundäre Aminogruppen oder tertiäre Aminogruppen sind oder bei dem eine Aminogruppe eine sterisch gehinderte sekundäre Aminogruppe und die andere Aminogruppe eine

tertiäre Aminogruppe ist, wobei der Grad der sterischen Hinderung so ist, daß der kumulative $^{-}E_s$-Wert (Taft's sterische Hinderungskonstante) größer als 1,75 ist.

2. Verfahren nach Anspruch 1, dadurch gekennzeichnet, daß der normalerweise gasförmige Strom außerdem $CO_2$ enthält, $H_2S$ selektiv aus dem Strom absorbiert wird und der Diaminoether einen $pK_a$-Wert bei 20°C von größer als 8,6 besitzt.

3. Verfahren nach Anspruch 1 oder 2, dadurch gekennzeichnet, daß die Gesamtkonzentration des Diaminoethers in der Absorptionslösung im Bereich von etwa 0,1 bis 6 Mol je 1 liegt, das Verfahren bei einer Temperatur im Bereich von etwa 20 bis 100°C und einem Druck im Bereich von 0,4 bis 140,6 $kg/cm^2$ durchgeführt wird, die Absorptionslösung durch Erhitzen bei einem Druck von 0,07 bis etwa 3,5 $kg/cm^2$ auf eine Temperatur von 50 bis etwa 170°C regeneriert wird und die Lösung von dem absorbierten $H_2S$ gestrippt wird.

4. Verfahren nach einem der Ansprüche 1 bis 3, dadurch gekennzeichnet, daß der Diaminoether folgende Formel besitzt:

$$N_1-\underset{R_3}{\overset{R_2}{(C)}}_m-[O-\underset{R_5}{\overset{R_4}{(C)}}_n]_o-O-\underset{R_7}{\overset{R_6}{(C)}}_p-N_2,$$

in der, wenn der Diaminoether ein Di-tertiäraminoether ist, $N_1$ und $N_2$ durch die Formeln:

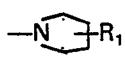 und 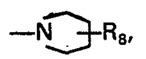

wiedergegeben werden, worin $R_1$ und $R_8$ jeweils unabhängig voneinander Wasserstoff oder ein Hydroxyl-, Alkyl-, Hydroxyalkyl-, Cycloalkyl-, Hydroxycycloalkyl-, Alkoxy- oder Hydroxyalkoxyrest sind, $R_2$, $R_3$, $R_4$, $R_5$, $R_6$ und $R_7$ jeweils unahängig Wasserstoff oder ein $C_1$- oder $C_2$-Alkyl oder $C_1$- oder $C_2$-Hydroxyalkylrest sind, m, n und p positive Zahlen von 2 bis 5 sind und o eine positive Zahl von 1 bis 4 ist, wenn der Diaminoether ein Di-sekundäraminoether ist, $N_1$ und $N_2$ wiedergegeben werden durch die Formeln:

$$-NH-R_1 \text{ und } -NH-R_8,$$

worin $R_1$ und $R_8$ jeweils unabhängig ein primärer Alkylrest mit 1 bis 8 Kohlenstoffatomen, ein primärer Hydroxyalkylrest mit 2 bis 8 Kohlenstoffatomen oder ein sekundärer oder tertiärer Alkyl- oder Hydroxyalkyl- oder Cycloalkyl- oder Hydroxycycloalkylrest mit 3 bis 8 Kohlenstoffatomen sind, $R_2$, $R_3$, $R_4$, $R_5$, $R_6$ und $R_7$ jeweils unabhängig Wasserstoff oder ein $C_1$—$C_4$-Alkyl- oder $C_1$—$C_4$-Hydroxyalkylrest sind mit der Maßgabe, daß, wenn die direkt an das Stickstoffatom gebundenen Kohlenstoffatome von $R_1$ und $R_8$ primär sind, $R_2$, $R_3$, $R_6$ und $R_7$ alle Alkyl- oder Hydroxyalkylreste sind und, wenn die direkt an das Stickstoffatom gebundenen Kohlenstoffatome von $R_1$ und $R_8$ sekundär sind, mindestens einer von $R_2$ oder $R_3$ und mindestens einer von $R_6$ oder $R_7$ Alkyl- oder Hydroxyalkylreste sind, m, n und p positive Zahlen von 2 bis 4 sind und o entweder 0 oder eine positive Zahl von 1 bis 10 ist, und, wenn der Diaminoether ein Disekundär- und -tertiäraminoether ist, $N_1$ und $N_1$ wiedergegeben werden durch die Formel

$-NH-R_1$ und 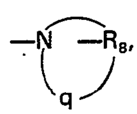

worin $R_1$ und $R_8$ jeweils unabhängig voneinander ein primärer Alkylrest mit 1 bis 8 Kohlenstoffatomen, ein primärer Hydroxyalkylrest mit 2 bis 8 Kohlenstoffatomen oder ein sekundärer oder tertiärer Alkyl- oder Hydroxyalkyl- oder Cycloalkyl- oder Hydroxycycloalkylrest mit 3 bis 8 Kohlenstoffatomen sind und $R_8$, der gegebenenfalls einen oder mehrere Substituenten an dem stickstoffhaltigen heterocyclischen Ring bedeuten kann, auch eine Hydroxylgruppe sein kann, q die Zahl der Kohlenstoffatome in dem heterocyclischen Ring bedeutet und eine positive Zahl von 3 bis 8 ist, $R_2$, $R_3$, $R_4$, $R_5$, $R_6$ und $R_7$ jeweils unabhängig Wasserstoff oder ein $C_1$—$C_4$-Alkyl- oder $C_1$—$C_4$-Hydroxyalkylrest sind mit der Maßgabe, daß, wenn das direkt an das Stickstoffatom gebundene Kohlenstoffatom von $R_1$ primär ist, sowohl $R_2$ als auch $R_3$ Alkyl- oder Hydroxylalkylreste sind und, wenn das direkt an das Stickstoffatom gebundene Kohlenstoffatom von $R_1$ sekundär ist, mindestens einer von $R_2$ oder $R_3$ ein Alkyl- oder Hydroxyalkylrest ist, m, n und positive Zahlen von 2 bis 4 sind und o entweder 0 oder eine positive Zahl von 1 bis 10 ist.

5. Verfahren nach einem der Ansprüche 1 bis 4, dadurch gekennzeichnet, daß der Diaminoether 1,2 - bis - (3 - Pyrrolidinyl - n - propoxy)ethan, 1,2 - bis - (t - Butylaminoethoxy)ethan oder 1 - (Pyrrolidinylethoxy) - 2 - (t - butylaminoethoxy)ethan ist.

6. Für das Verfahren gemäß einem der vorangehenden Ansprüche geeignete Absorptions lösung, die ein Lösungsmittel und einen Diaminoether enthält, bei dem beide Aminogruppen entweder sterisch gehinderte sekundäre Aminogruppen sind oder bei dem eine Aminogruppe eine sterisch gehinderte sekundäre Aminogruppe und die andere Aminogruppe eine tertiäre Aminogruppe ist, wobei der Grad der

sterischen Hinderung so ist, daß der kumulative $^{-}E_s$-Wert (Taft's sterische Hinderungskonstante) größer als 1,75 ist.

7. Lösung nach Anspruch 6, dadurch gekennzeichnet, daß das Lösungsmittel ein physikalisches Absorptionsmittel ist.

8. Diaminoetherzusammensetzung mit der folgenden Formel:

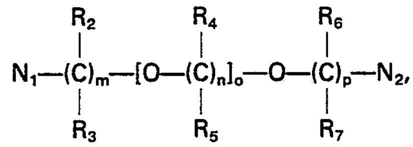

in der, wenn der Diaminoether ein Di-sekundäraminoether ist, $N_1$ und $N_2$ wiedergegeben werden durch die Formeln:

$$—NH—R_1 \text{ und } —NH—R_8,$$

worin $R_1$ und $R_8$ jeweils unabhängig ein primärer Alkylrest mit 1 bis 8 Kohlenstoffatomen, ein primärer Hydroxyalkylrest mit 2 bis 8 Kohlenstoffatomen oder ein sekundärer oder tertiärer Alkyl- oder Hydroxyalkyl- oder Cycloalkyl- oder Hydroxycycloalkylrest mit 3 bis 8 Kohlenstoffatomen sind, $R_2$, $R_3$, $R_4$, $R_5$, $R_6$ und $R_7$ jeweils unabhängig Wasserstoff oder ein $C_1$—$C_4$-Alkyl- oder $C_1$—$C_4$-Hydroxyalkylrest mit der Maßgabe sind, daß, wenn die direkt an das Stickstoffatom gebundenen Kohlenstoffatome von $R_1$ und $R_8$ primär sind, $R_2$, $R_3$, $R_6$ und $R_7$ alle Alkyl- oder $C_1$—$C_4$-Hydroxyalkylreste sind und, wenn die direkt an das Stickstoffatom gebundenen Kohlenstoffatome von $R_1$ und $R_8$ sekundär sind, mindestens einer von $R_2$ oder $R_3$ und mindestens einer von $R_6$ oder $R_7$ Alkyl- oder Hydroxyalkylreste sind, m, n und p positive Zahlen von 2 bis 4 sind und o endweder 0 oder eine positive Zahl von 1 bis 10 ist, und wenn der Diaminoether ein Disekundär- und Tertiäraminoether ist, $N_1$ und $N_2$ wiedergegeben werden durch die Formeln:

—NH —$R_1$ und 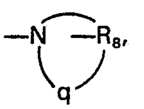

worin $R_1$ und $R_8$ jeweils unabhängig ein primärer Alkylrest mit 1 bis 8 Kohlenstoffatomen, ein primärer Hydroxyalkylrest mit 2 bis 8 Kohlenstoffatomen oder ein sekundärer oder tertiärer Alkyl- oder Hydroxyalkyl- oder Cycloalkyl- oder Hydroxycycloalkylrest mit 3 bis 8 Kohlenstoffatomen sind und $R_8$, der gegebenenfalls einen oder mehrere Substituenten am stickstoffhaltigen heterocyclischen Ring bedeuten kann, auch eine Hydroxylgruppe sein kann, q die Zahl der Kohlenstoffatome in dem heterocyclischen Ring bedeutet und eine positive Zahl von 3 bis 8 ist, $R_2$, $R_3$, $R_4$, $R_5$, $R_6$ und $R_7$ jeweils unabhängig Wasserstoff oder ein $C_1$—$C_4$-Alkyl- oder $C_1$—$C_4$-Hydroxyalkylrest sind mit der Maßgabe, daß, wenn das direkt an das Stickstoffatom gebundene Kohlenstoffatom von $R_1$ primär ist, sowohl $R_2$ als auch $R_3$ Alkyl- oder Hydroxyalkylreste sind und, wenn das direkt an das Stickstoffatom gebundene Kohlenstoffatom von $R_1$ sekundär ist, mindestens einer von $R_2$ oder $R_3$ ein Alkyl- oder Hydroxyalkylrest ist, m, n und p positive Zahlen von 2 bis 4 sind und o entweder 0 oder eine positive Zahl von 1 bis 10 ist.

9. Diaminoether nach Anspruch 8, dadurch gekennzeichnet, daß er 1,2 - bis - (t - Butylaminoethoxy)ethan oder 1 - (Pyrrolidinylethoxy) - 2 - (t - Butylaminoethoxy)ethan ist.

10. Verfahren zur Herstellung des Di-sekundäraminoethers nach Anspruch 8, dadurch gekennzeichnet, daß man ein primäres Amin mit einem di-(Haloalkoxy)alkan oder einem bis-Sulfonatester eines Glykolethers bei erhöhter Temperatur zur Reaktion bringt.

11. Verfahren zur Herstellung des Di-Sekundär- und -tertiäraminoethers nach Anspruch 8, dadurch gekennzeichnet, daß man

(a) eine heterocyclische Verbindung mit 4 bis 9 Ringatomen mit einem Haloalkoxyalkanol oder einem Haloalkoxyalkoxyalkanol bei erhöhter Temperatur kontaktiert,

(b) das Produkt aus Stufe (a) mit einem Thionylhalogenid umsetzt und

(c) das Produkt aus Stufe (b) bei erhöhter Temperatur mit einem primären Alkylamin umsetzt.

**Revendications**

1. Procédé pour éliminer $H_2S$ d'un courant normalement gazeux à l'aide d'une solution absorbante comprenant un solvant et un diaminoéther dans lequel les deux groupes amino sont soit des groupes amino secondaires à empêchement stérique ou bien sont des groupes amino tertiaires, ou dans lequel un groupe amino est un groupe amino secondaire à empêchement stérique et l'autre groupe amino est un groupe amino tertiaire, le degré d'empêchement stérique étant tel que la valeur cumulative $^{-}E_s$ (constant d'empêchement stérique de Taft) soit supérieure à 1,75.

2. Procédé selon la revendication 1, dans lequel le courant normalement gazeux contient aussi $CO_2$, le

$H_2S$ est sélectivement absorbé à partir du courant et le diaminoéther a une valeur de $pK_a$ à 20°C supérieure à 8,6.

3. Procédé selon la revendication 1 ou 2, dans lequel la concentration totale du diaminoéther dans la solution absorbante se situe entre environ 0,1 et 6 moles per litre, le procédé est mis en oeuvre à une température comprise entre environ 20 et 100°C et à une pression comprise entre 0,4 et 140,6 kg/cm$^2$, la solution absorbante est régénérée par chauffage à une température comprise entre 50 et environ 170°C et à une pression comprise entre 0,07 et environ 3,5 kg/cm$^2$, et la solution est débarrassée de l'$H_2S$ qu'elle avait absorbé.

4. Procédé selon l'une quelconque des revendications 1 à 3, dans lequel le diaminoéther répond à la formule suivante:

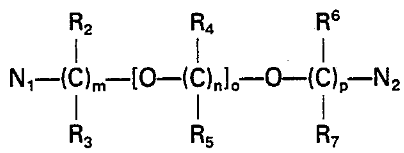

dans laquelle, si le diaminoéther est un di-tertioaminoéther, $N_1$ et $N_2$ sont représentés par les formules:

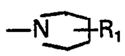 et 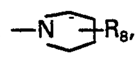, respectivement,

dans lesquelles $R_1$ et $R_8$ représentent chacun, indépendamment, un atome d'hydrogène ou un radical hydroxyle, alkyle, hydroxyalkyle, cycloalkyle, hydroxycycloalkyle, alkoxy ou hydroxyalkoxy, $R_2$, $R_3$, $R_4$, $R_5$, $R_6$ et $R_7$ représentent chacun, indépendamment, un atome d'hydrogène ou un radical alkyle en $C_1$ ou $C_2$ ou hydroxyalkyle en $C_1$ ou $C_2$, m, n et p sont des nombres entiers positifs valant de 2 à 5, et o est un nombre entier positif valant de 1 à 4; dans laquelle si le diaminoéther est un di-(amino secondaire) éther, $N_1$ et $N_2$ sont représentés par les formules:

$$—NH\ —R_1 \text{ et } —NH\ —R_8, \text{ respectivement,}$$

dans lesquelles $R_1$ et $R_8$ représentent chacun, indépendamment, un radical alkyle primaire ayant 1 à 8 atomes de carbone, un radical hydroxyalkyle primaire 2 à 8 atomes de carbone, ou un radical alkyle ou hydroxyalkyle ou cycloalkyle ou hydroxycycloalkyle secondaire ou tertiaire ayant 3 à 8 atomes de carbone, $R_2$, $R_3$, $R_4$, $R_5$, $R_6$ et $R_7$ représentent chacun, indépendamment, un atome d'hydrogène ou un radical alkyle en $C_1$ à $C_4$ ou hydroxyalkyle en $C_1$ à $C_4$, à la condition que, lorsque les atomes de carbone de $R_1$ et $R_8$ directement liés à l'atome d'azote sont primaires, $R_2$, $R_3$, $R_6$ et $R_7$ soient tous des radicaux alkyles ou hydroxyalkyles et, quand les atomes de carbone de $R_1$ et $R_8$ directement liés à l'atome d'azote sont secondaires, au moins l'un des $R_2$ ou $R_3$ et au moins l'un des $R_6$ ou $R_7$ représentent des radicaux alkyles ou hydroxyalkyles, m, n et p sont des nombres entiers positifs valant 2 à 4, et o est 0 ou est un nombre entier positif valant 1 à 10; et dans laquelle si le diaminoéther est un di(amino secondaire et tertiaire)-éther. $N_1$ et $N_2$ sont représentés par les formules:

—NH —$R_1$ et 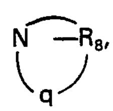, respectivement,

dans lesquelles $R_1$ et $R_8$ représentent chacun, indépendamment, un radical alkyle primaire ayant 1 à 8 atomes de carbone, un radical hydroxyalkyle primaire ayant 2 à 8 atomes de carbone ou un radical alkyle ou hydroxyalkyle ou cycloalkyle ou hydroxycycloalkyle secondaire ou tertiaire, ayant 3 à 8 atomes de carbone, et $R_8$, représentant un ou plusieurs substituants facultatifs sur le noyau hétérocyclique azoté, peut aussi être un groupe hydroxyle, q représente le nombre des atomes de carbone du noyau hétérocyclique et est un nombre entier positif valant de 3 à 8, $R_2$, $R_3$, $R_4$, $R_5$, $R_6$ et $R_7$ représentent chacun, indépendamment, un atome d'hydrogène ou un radical alkyle en $C_1$ à $C_4$ ou hydroxyalkyle en $C_1$ à $C_4$, à la condition que, lorsque l'atome de carbone de $R_1$ directement lié à l'atome d'azote est primaire, $R_2$ et $R_3$ représentent tous deux des radicaux alkyles ou hydroxyalkyles, et lorsque l'atome de carbone de $R_1$ directement lié à l'atome d'azote est secondaire, au moins l'un des $R_2$ ou $R_3$ représentent un radical alkyle ou hydroxyalkyle, m, n et p sont des nombres entiers positifs valant 2 à 4, et o est 0 ou est un nombre entier positif valant 1 à 10.

5. Procédé selon l'une quelconque des revendications 1 à 4, dans lequel le diaminoéther est le 1,2 - bis - (3 - pyrrolidinyl - n - propoxy)éthane, le 1,2 - bis - (tertiobutylaminoéthoxy)éthane ou le 1 - (pyrrolidinyléthoxy - 2 - (t - butyl - amino - éthoxy)éthane.

6. Solution absorbante utilisable pour le procédé selon l'une quelconque des revendications précédentes, qui comprend un solvant et un diaminoéther dans lequel les deux groupes amino sont soit des groupes amino secondaires à empêchement stérique ou dans lequel un groupe amino est un groupe amino secondaire à empêchement stérique et l'autre groupe amino est un groupe amino tertiaire, le degré

d'empêchement stérique étant tel que la valeur de $^{-}E_s$ cumulative (constante d'empêchement stérique de Taft) soit supérieure à 1,75.

7. Solution selon la revendication 6, dans laquelle le solvant est un absorbant physique.

8. Composition de diaminoéther ayant la formule suivante:

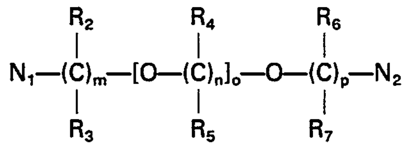

dans laquelle, si le diaminoéther est un di(amino secondaire)-éther, $N_1$ et $N_2$ sont représentés par les formules:

$$—NH\ —R_1 \text{ et } —NH\ —R_8, \text{ respectivement}$$

dans lesquelles $R_1$ et $R_8$ représentent chacun, indépendamment, un radical alkyle primaire ayant 1 à 8 atomes de carbone, un radical hydroxyalkyle primaire ayant 2 à 8 atomes de carbone ou un radical alkyle ou hydroxyalkyle ou cycloalkyle ou hydroxycycloalkyle secondaire ou tertiaire ayant 3 à 8 atomes de carbone, $R_2$, $R_3$, $R_4$, $R_5$, $R_6$ et $R_7$ représentent chacun, indépendamment, un atome d'hydrogène ou un radical alkyle en $C_1$ à $C_4$ ou hydroxyalkyle en $C_1$ à $C_4$, à la condition que, lorsque les atomes de carbone de $R_1$ et $R_8$ directement liés à l'atome d'azote sont primaires, $R_2$, $R_3$, $R_6$ et $R_7$ soient tous des radicaux alkyles ou hydroxyalkyles en $C_1$ à $C_4$ et, lorsque les atomes de carbone de $R_1$ et $R_8$ directement liés à l'atome d'azote sont secondaires, au moins l'un des $R_2$ ou $R_3$ et au moins l'un des $R_6$ ou $R_7$ représentent un radical alkyle ou hydroxyalkyle, m, n et p sont des nombres entiers positifs valant 2 à 4, et o est 0 ou est un nombre entier positif valant 1 à 10; et dans laquelle si le diaminoéther est un di(amino secondaire et tertiaire)-éther, $N_1$ et $N_2$ sont représentés par les formules:

—NH— $R_1$ et 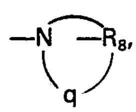 , respectivement,

dans lesquelles $R_1$ et $R_8$ représentent chacun, indépendamment, un radical alkyle primaire ayant 1 à 8 atomes de carbone, un radical hydroxyalkyle primaire ayant 2 à 8 atomes de carbone ou un radical alkyle ou hydroxyalkyle ou cycloalkyle ou hydroxycycloalkyle secondaire ou tertiaire ayant 3 à 8 atomes de carbone, et $R_8$, représentant un ou plusieurs substituants facultatifs sur le noyau hétérocyclique azoté, peut aussi être un groupe hydroxyle, q représente le nombre des atomes de carbone du noyau hétérocyclique et est un nombre entier positif valant 3 à 8, $R_2$, $R_3$, $R_4$, $R_5$, $R_6$ et $R_7$ représentent chacun, indépendamment, un atome d'hydrogène ou un radical alkyle en $C_1$ à $C_4$ ou hydroxyalkyle en $C_1$ à $C_4$, à la condition que, lorsque l'atome de carbone de $R_1$ directement lié à l'atome d'azote est primaire, les deux radicaux $R_2$ et $R_3$ soient des radicaux alkyles ou hydroxyalkyles et, lorsque l'atome de carbone de $R_1$ directement lié à l'atome d'azote est secondaire, au moins l'un des $R_2$ ou $R_3$ représente un radical alkyle ou hydroxyalkyle, m, n et p sont des nombres entier positifs valant 2 à 4, et o est 0 ou est un nombre entier positif valant 1 à 10.

9. Diaminoéther selon la revendication 8, qui est le 1,2 - bis - (tertiobutylaminoéthoxy)éthane ou le 1 - (pyrrolidinyléthoxy) - 2 - (tertiobutylaminoéthoxy)éthane.

10. Procédé pour préparer le di(amino secondaire)éther selon la revendication 8, qui comprend la réaction d'une amine primaire avec un di(halogénoalkoxy)alkane ou un bis-éther sulfonique d'un éther glycolique, à une température élevée.

11. Procédé pour préparer le di(amino secondaire et tertiaire)-éther selon la revendication 8, qui comprend:

(a) la mise en contact d'un composé hétérocyclique, ayant 4 à 9 atomes de cycle, avec un halogénoalkoxyalkanol ou un halogénoalkoxyalkoxyalkanol à une température élevée;

(b) la réaction du produit obtenu à l'étape (a) avec un halogénure de thionyle; et

(c) la réaction du produit obtenu à l'étape (b) avec une alkylamine primaire à une température élevée.

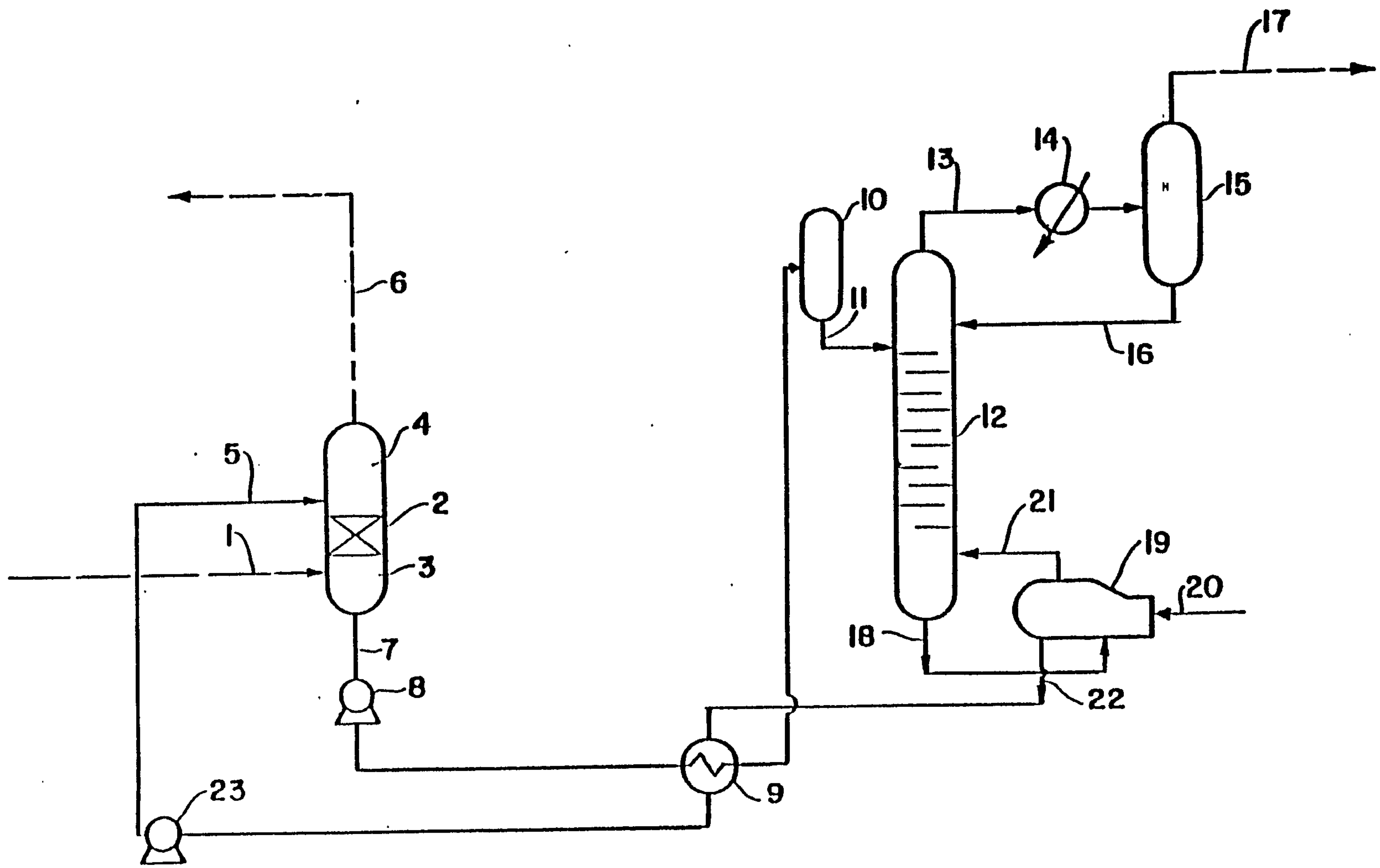


Absorption-regeneration unit for selective $H_2S$ removal

FIG. 1

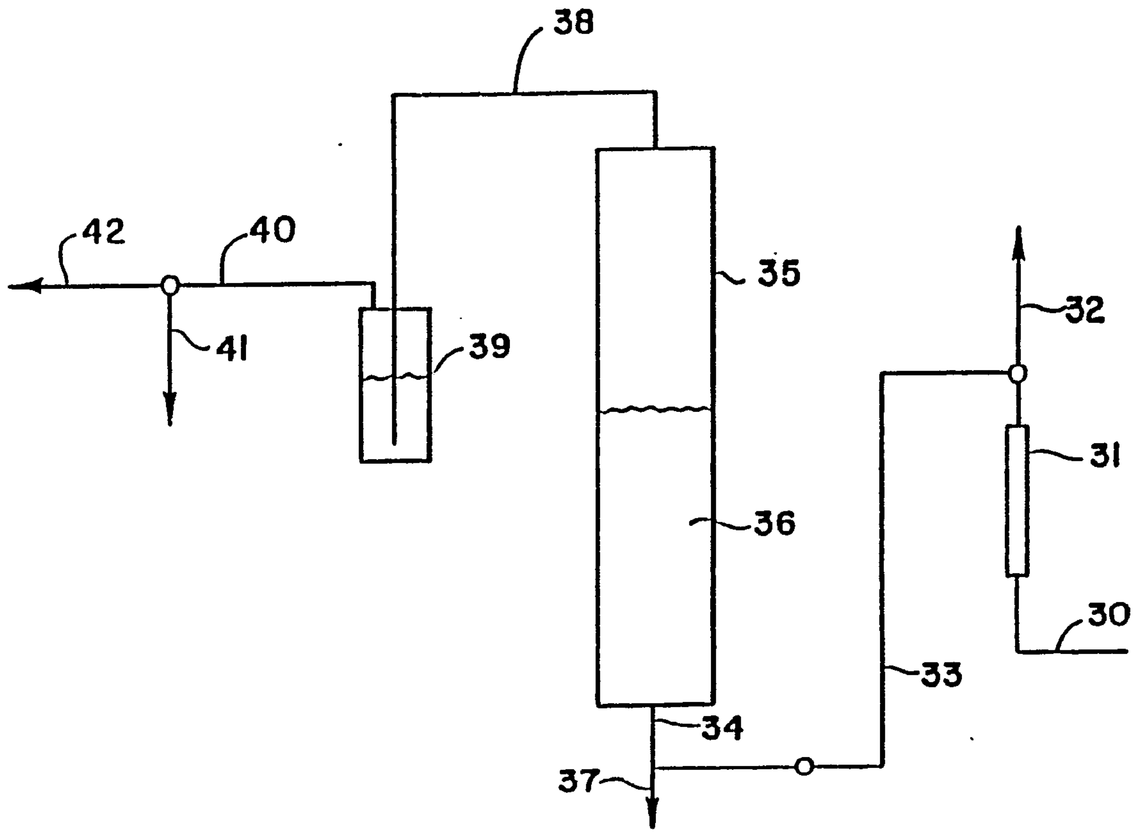


Sparged absorber unit for selective $H_2S$ removal

FIG. 2

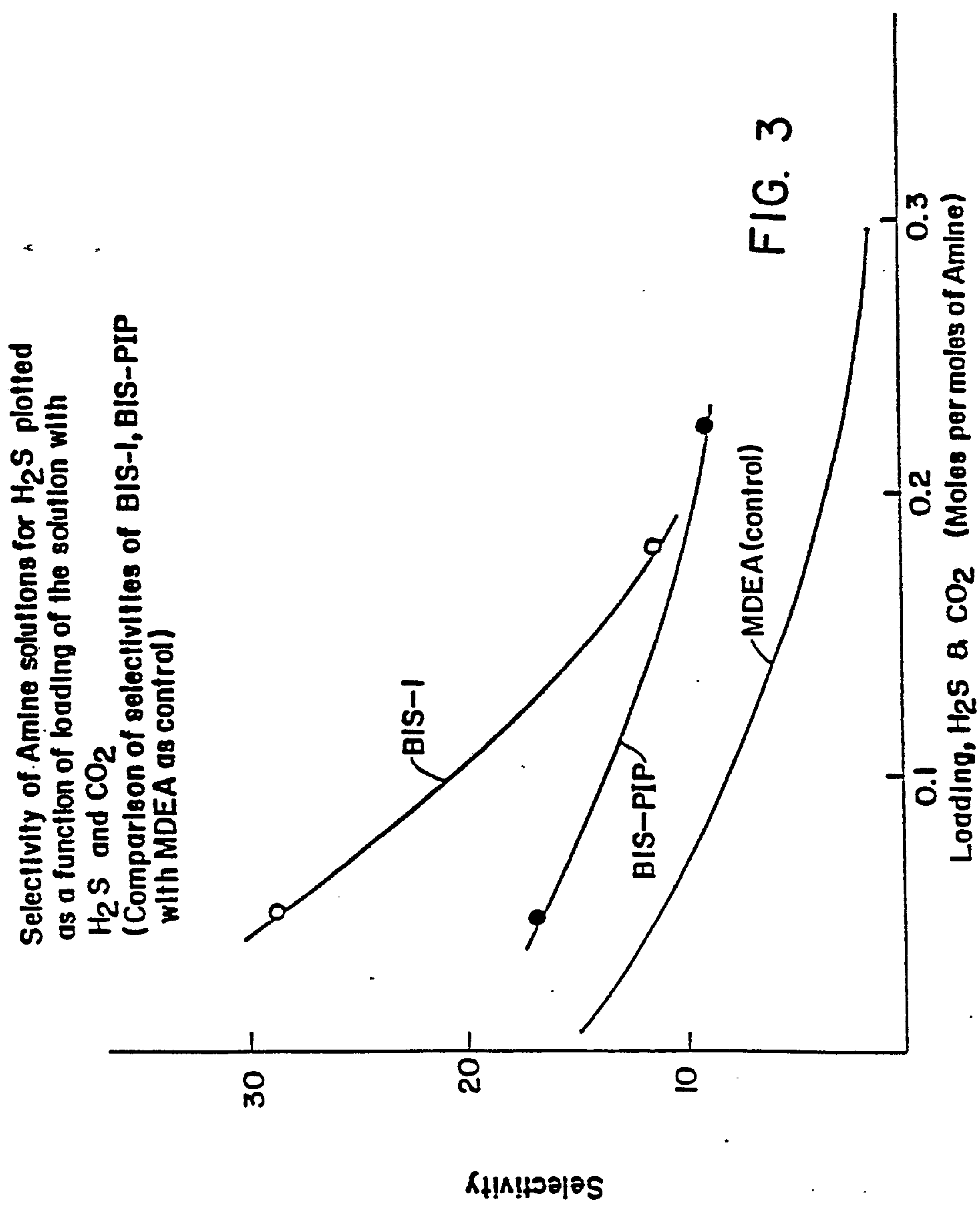
Selectivity of Amine solutions for $H_2S$ plotted as a function of loading of the solution with $H_2S$ and $CO_2$
(Comparison of selectivities of BIS-I, BIS-PIP with MDEA as control)
BIS-I
BIS-PIP
MDEA (control)
Selectivity
30
20
10
0.1
0.2
0.3
Loading, $H_2S$ & $CO_2$ (Moles per moles of Amine)

FIG. 3

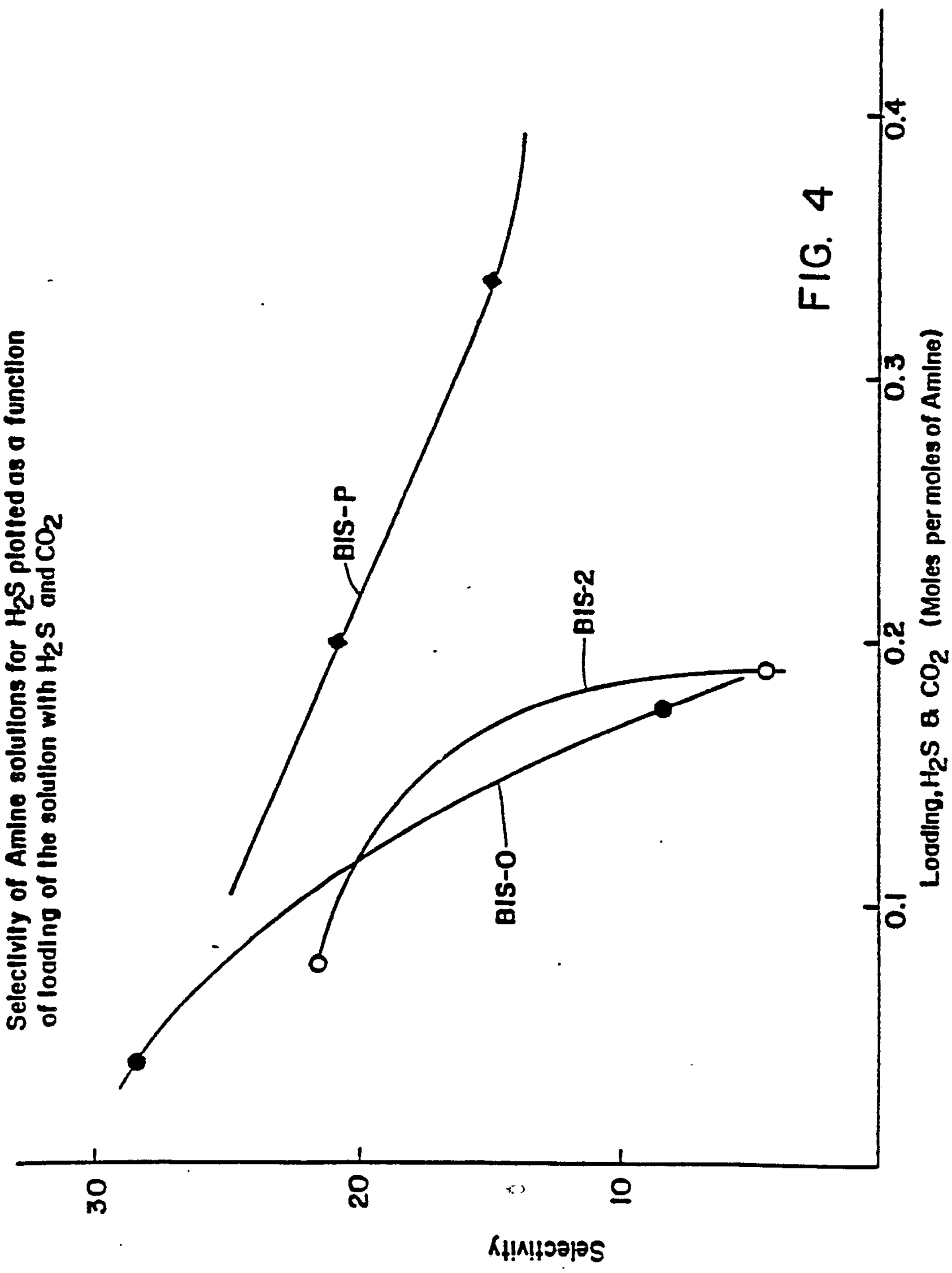

Selectivity of Amine solutions for $H_2S$ plotted as a function of loading of the solution with $H_2S$ and $CO_2$
BIS-P
BIS-2
BIS-O
30
20
10
Selectivity
0.1
0.2
0.3
0.4
Loading, $H_2S$ & $CO_2$ (Moles per moles of Amine)


FIG. 4

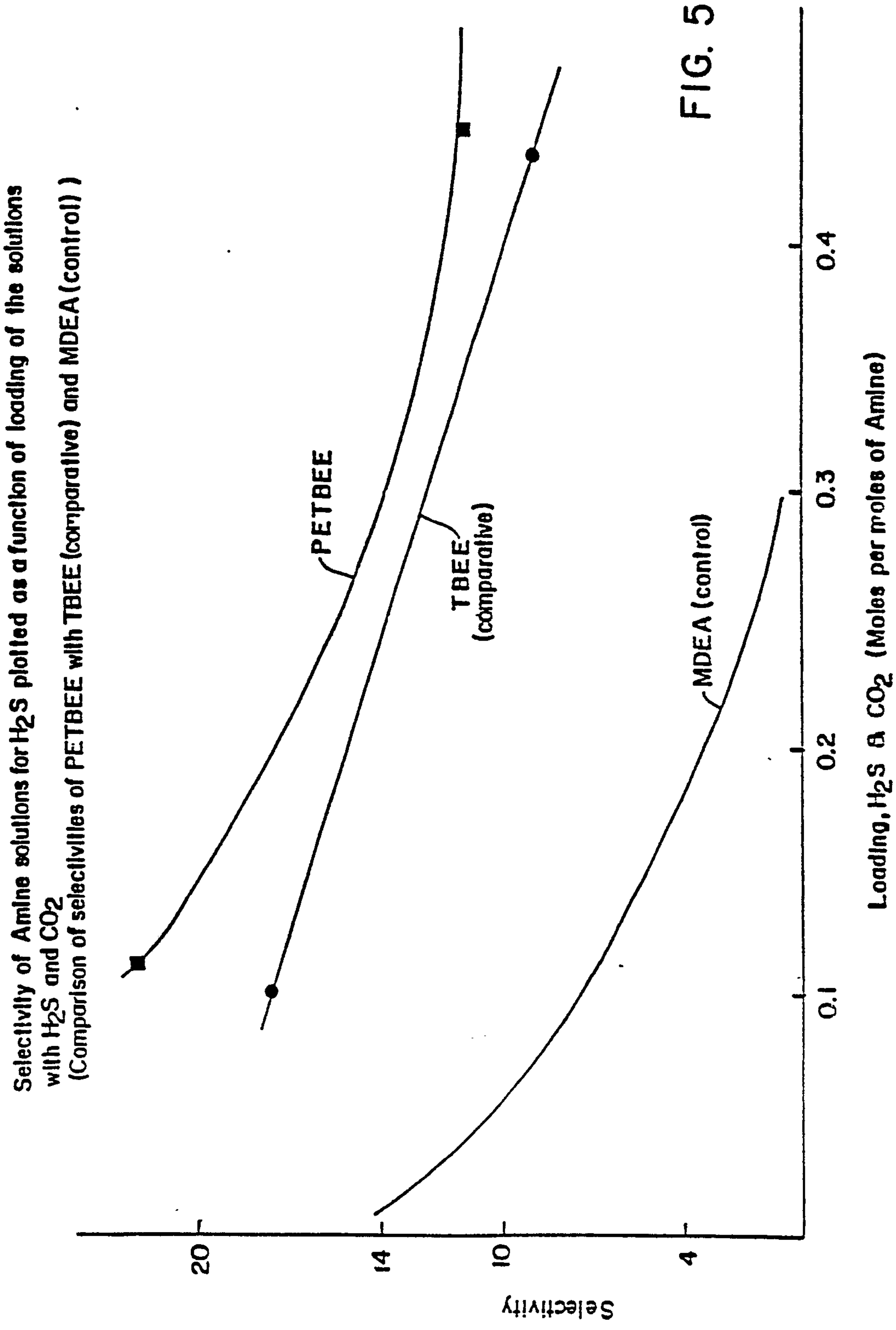
Selectivity of Amine solutions for $H_2S$ plotted as a function of loading of the solutions with $H_2S$ and $CO_2$
(Comparison of selectivities of PETBEE with TBEE (comparative) and MDEA (control) )
PETBEE
TBEE
(comparative)
MDEA (control)
Selectivity
20
14
10
4
0.1
0.2
0.3
0.4
Loading, $H_2S$ & $CO_2$ (Moles per moles of Amine)

FIG. 5

Selectivity of Amine solutions for $H_2S$ plotted as a function of loading of the solution with $H_2S$ and $CO_2$
(Comparison of selectivities of BIS-TB, TBEE (comparative) and MDEA and BIS-IP as controls)

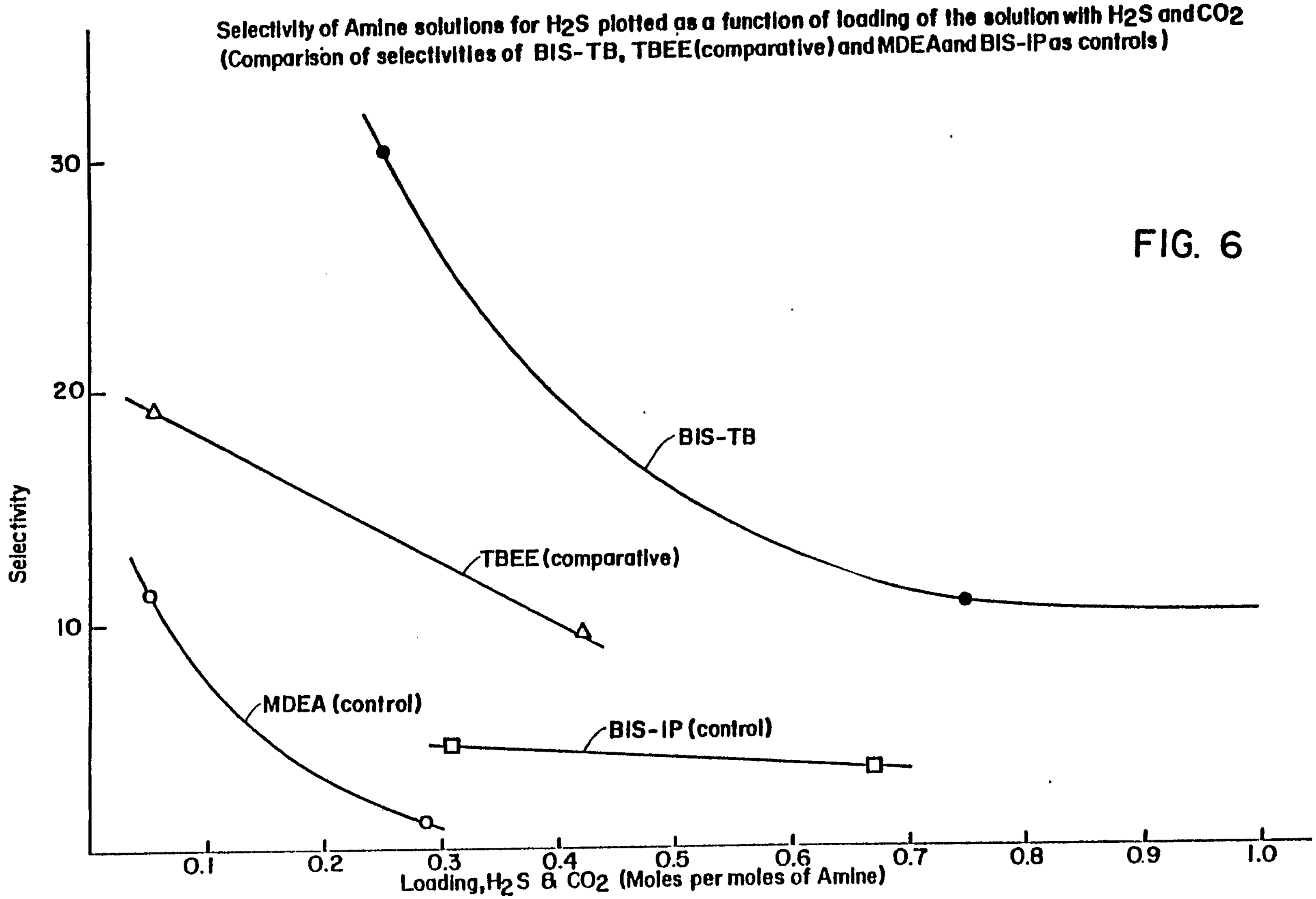


FIG. 6